# EUROPEAN PATENT SPECIFICATION

(11) **EP 1 728 505 B1**
(45) Date of publication and mention of the grant of the patent: **04.06.2014**
(21) Application number: 05727185.0
(22) Date of filing: 23.03.2005
(51) Int. Cl.: A61K 9/10, A61K 9/48, A61K 31/4178, A61K 47/02, A61K 47/08, A61K 47/10, A61K 47/14, A61K 47/34, A61K 47/44, A61P 31/18, C07D 403/12, A61K 9/107

(54) **PREPARATION WITH ELEVATED CONTENT**
ZUBEREITUNG MIT ERHÖHTEM GEHALT
PRÉPARATION EN TENEUR ÉLEVÉE

(30) Priority: 24.03.2004 JP 2004087032
(43) Date of publication of application: 06.12.2006
(73) Proprietor: Takeda Pharmaceutical Company Limited, Osaka-shi, Osaka 541-0045 (JP)
(72) Inventor: YOSHINARI, Tomohiro, TAKEDA PHARM. COMPANY LTD, Osaka-shi, Osaka; 5328686 (JP)
(74) Representative: Von Kreisler Selting Werner - Partnerschaft von Patentanwälten und Rechtsanwälten mbB
(86) International application number: PCT/JP2005/005239
(87) International publication number: WO 2005/089716

(56) References cited:
- EP-A- 1 182 195
- EP-A- 1 236 476
- WO-A-99/49848
- WO-A1-00/61113
- WO-A1-01/76582
- WO-A1-03/014105
- WO-A1-2005/070399
- CA-A1- 2 443 461
- JP-A- 2 121 929
- JP-A- 8 073 476
- JP-A- 60 208 927
- JP-A- 63 010 717
- JP-A- 63 150 221
- JP-A- 2003 335 776
- JP-A- 2003 512 312
- US-A- 6 054 136
- US-A1- 2003 086 948
- ALTRIA K D: "Background theory and applications of microemulsion electrokinetic chromatography", JOURNAL OF CHROMATOGRAPHY, ELSEVIER SCIENCE PUBLISHERS B.V, NL, vol. 892, no. 1-2, 15 September 2000 (2000-09-15), pages 171-186, XP004212062, ISSN: 0021-9673, DOI: 10.1016/S0021-9673(00)00088-1

## Description

### Technical Field

The present invention relates to a pharmaceutical composition for oral use comprising a medicinal compound in an elevated amount, and a preparation enclosing the same.

### Background Art

The Self Micro-Emulsifying Drug Delivery System (SMEDDS^{™}) is a drug delivery system developed by Gattefossé (France). This system is composed of three components, i.e., a surfactant, a cosurfactant and a lipid phase (refer to patent document 1 below). The present system is a compatible mixture consisting of these three components, which is characterized in that a microemulsion is spontaneously produced when water is added thereto as a fourth component. A well-known example of an application of this technology is Neoral^{™}, which is a Cyclosporin immunosuppressive preparation that is commercially available from Novartis.

On the other hand, in the present system, it is preferred to completely dissolve the medicinal compound in the three components, but the solubility of medicinal compounds in aqueous medium is often low, thus the content thereof is restricted. Therefore, the development of a method that can achieve a higher content has been a topic of investigation.

### [patent document 1] US 6054136

WO 01/28520 discloses microemulsion preconcentrates comprising an oily base, a surfactant and a water insoluble medicinal compound.

WO 03/014105 discloses the medicinal compounds used in the present invention.

### Disclosure of Invention

### Problems to be Solved by the Invention

An object of the present invention is to provide a composition for oral use which contains a medicinal compound, in particular, a hardly water-soluble or water-insoluble medicinal compound in an elevated amount and is excellent in absorbability of the medicinal compound via the digestive tract, and a preparation enclosing the composition.

### Means of Solving the Problems

The present invention provides:

A liquid transparent pharmaceutical composition comprising
(i) a polar solvent in an amount of 1 w/w% to 20 w/w%, wherein in the polar solvent is water,
(ii) an oily base,
(iii) a surfactant having an HLB of 14 or more, wherein the content (w/w%) of the surfactant is more than the content (w/w%) of the oily base,
(iv) a medicinal compound having a solubility of less than 0.1 mg/ml in water at 25°C, wherein the content of the medicinal compound in the composition is 1 w/w% or more and wherein the content of the medicinal compound is an amount exceeding the solubility thereof in the mixture of the oily base and surfactant, wherein the medicinal compound is a salt of the compound represented by formula (I) : 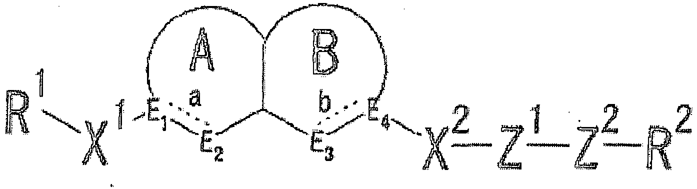
   wherein, R¹ denotes an optionally substituted 5- to 6-membered ring,
   X¹ denotes a bond or a divalent group wherein the number of atoms constituting the straight-chain moiety is 1 to 4, ring A denotes an optionally substituted 5- or 6-membered ring, and ring B denotes an optionally substituted 8- to 10-membered ring,
   E₁ and E₄ each denote an optionally substituted carbon atom or an optionally substituted nitrogen atom,
   E₂ and E₃ each denote an optionally substituted carbon atom, optionally substituted nitrogen atom, optionally oxidized sulfur atom or oxygen atom,
   a and b each denote a single bond or a double bond,
   X² denotes a divalent group wherein the number of atoms constituting the straight chain moiety is 1 to 4,
   Z¹ denotes a bond or a divalent cyclic group,
   Z² denotes a bond or a divalent group,
   R² denotes (1) an optionally substituted amino group wherein the nitrogen atom may be converted into a quaternary ammonium or oxide, (2) an optionally substituted nitrogen-containing heterocyclic group that may comprise sulfur atoms or oxygen atoms as ring constituent atoms, wherein the nitrogen atom may be converted into a quaternary ammonium or oxide, (3) a group represented by the formula: 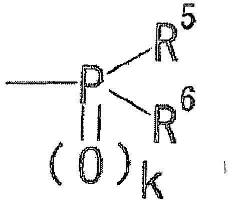
   wherein, k denotes 0 or 1, and when k is 0, the phosphorus atom can form a phosphonium salt, R⁵ and R⁶ each denote an optionally substituted hydrocarbon group, optionally substituted hydroxyl group, or optionally substituted amino group, and R⁵ and R⁶ may be bonded together to form a cyclic group along with an adjacent phosphorus atom, (4) an optionally substituted amidino group, or (5) an optionally substituted guanidino group.

Preferred embodiments of the invention are apparent from the dependent claims.

### Brief Description of Drawings

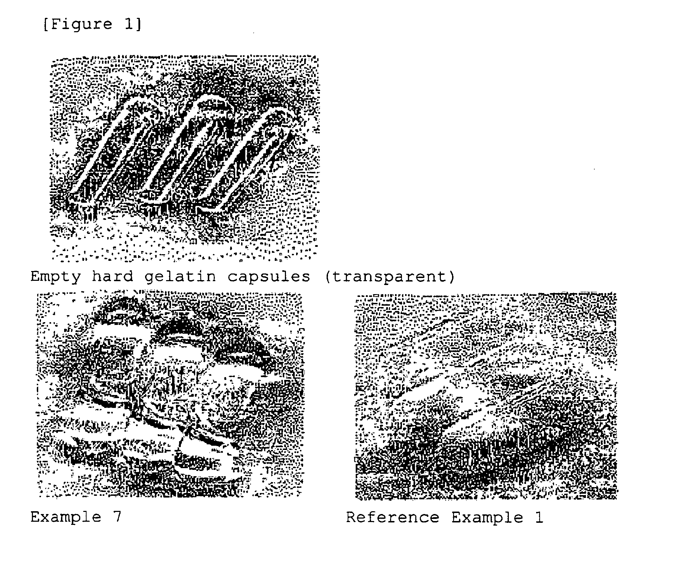

Figure 1 is a photograph showing a comparison of the appearance of the capsules in which the compositions obtained in Example 7 and Reference Example 1 were encapsulated.

### Best Mode for Carrying Out the Invention

The pharmaceutical composition of the present invention contains the medicinal compound as defined in claim 1 which is a hardly water-soluble or a water-insoluble medicinal compound in an elevated amount, and when administered orally, a stable microemulsion is formed in the digestive tract wherein fine particles containing the medicinal compound are dispersed, and thus, there are provided a composition for oral use which is excellent in the absorbability of the medicinal compound via the digestive tract and has a high bioavailability thereof, and a preparation for oral use enclosing the composition.

The pharmaceutical composition of the present invention is a transparent liquid composition which comprises a medicinal compound, an oily base, a surfactant and a polar solvent that is a poor solvent for the medicinal compound, and, wherein the content of the medicinal compound exceeds the solubility thereof in the mixture of the oily base and surfactant.

The term "poor solvent" in the "polar solvent that is a poor solvent for the medicinal compound" used herein refers to a solvent that has a low capacity for dissolving the medicinal compound, for example, a solvent having a solubility of the medicinal compound in the solvent such that 30 mL or more of the solvent is required in order to dissolve 1 mL or 1 g of solute, and preferably 100 mL or more of the solvent is required in order to dissolve 1 g or 1 mL of solute. According to the present invention the polar solvent that is a poor solvent for the medicinal compound is water.

The content of the polar solvent in the composition of the present invention is 1 w/w% to 20 w/w% with respect to the entire composition.

Examples of oily bases in the present invention include glycerin fatty acid ester, soy oil, olive oil, orange oil, hydrogenated oils, sesame oil, camellia oil, corn oil, paraffin, rapeseed oil, cocoanut oil, Vaseline, eucalyptus oil, peanut oil, wheat germ oil, triethyl citrate, triacetin, oleic acid, lauric acid, capric acid, caprylic acid, linolic acid, linoleic acid, palmitic acid, and the like. Examples of glycerin fatty acid esters include glycerin mono-fatty acid ester, glycerin di-fatty acid ester, and glycerin tri-fatty acid ester; specifically, glycerin medium-chain fatty acid esters such as caprylic acid/capric acid triglycerides and the like.

Among these, caprylic acid/capric acid triglycerides which are glycerin tri-medium chain fatty acid esters are preferred.

The content of the oily base in the composition of the present invention is 1 w/w% to 50 w/w%, preferably 5 w/w% to 30 w/w% with respect to the entire composition.

Nonionic surfactants and surfactants derived from natural materials and the like may be used for the surfactant in the present invention. Examples of the above nonionic surfactants that may be used include glycerin fatty acid esters, fatty acid-ethylene oxide adducts, higher alcohol-ethylene oxide adducts, alkyl phenol-ethylene oxide adducts, polyhydric alcohol fatty acid ester-ethylene oxide adducts, higher alkylamine-ethylene oxide adducts, fatty acid amide-ethylene oxide adducts, oil-ethylene oxide adducts, pentaerythritol fatty acid esters, polyhydric alcohol alkyl ethers, fatty acid amides of alkanolamines, and the like. Specifically, used preferably are sorbitol and sorbitan fatty acid esters, polyoxyethylene sorbitan fatty acid esters, polyethylene glycol fatty acid esters, sucrose fatty acid esters, polyoxyethylenated glycerin fatty acid esters, polyoxyethylene castor oil (polyethoxylated castor oil), polyoxyethylene-hydrogenated castor oil (polyethoxylated hydrogenated castor oil), polyoxyethylene polypropylene glycol copolymer, glycerin fatty acid ester, polyglycerin fatty acid ester, and the like.

Examples of natural-derived surfactants that may be used include lecithin phospholipids such as egg yolk lecithin (trade name: PL-100H, Kewpie), soy lecithin (trade name: Lecinol S-10, Nikko Chemicals), and the like.

In addition, the surfactant in the present invention has an HLB of 14 or more.

The content of the surfactant in the composition of the present invention is 10 w/w% to 90 w/w%, preferably 30 w/w% to 90 w/w% with respect to the total amount of the composition. In addition, the content of the surfactant in the composition of the present invention is more than the content of the oily base.

The present invention has, for the hardly water-soluble or water-insoluble medicinal compound, superior effects in terms of increasing absorbability in the digestive tract and improving bioavailability of the medicinal compound when administered orally.

The terms "hardly water-soluble or water-insoluble" in the "hardly water-soluble or water-insoluble medicinal compound" mentioned above denotes a solubility of less than 0.1 mg/mL in water at 25°C. The solubility may be measured by a common method.

The medicinal compound of the present invention is preferably a compound wherein the pKa or pKb of the free form is 6 or less, preferably 5 or less.

The content of the medicinal compound in the composition of the present invention is 1 w/w% or more, preferably 5 w/w% or more with respect to the total amount of the composition.

Examples of the "5- to 6-membered ring" in the "optionally substituted 5- to 6-membered ring" represented by R¹ in formula (I) above include groups formed by removing one hydrogen atom from 6-membered aromatic hydrocarbons such as benzene; 5- to 6-membered aliphatic hydrocarbons such as cyclopentane, cyclohexane, cyclopentene, cyclohexene, cyclopentadiene, cyclohexadiene; 5- to 6-membered aromatic heterocyclic ring having 1 to 4 of 1 to 2 kinds of hetero atoms selected from nitrogen atom, sulfur atom and oxygen atom such as furan, thiophene, pyrrole, imidazoles, pyrazole, thiazole, oxazole, isothiazole, isoxazole, tetrazole, pyridine, pyrazine, pyrimidine, pyridazine, triazole; and 5- to 6-membered non-aromatic heterocyclic ring having 1 to 4 of 1 to 2 kinds of hetero atoms selected from nitrogen atom, sulfur atom and oxygen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, and the like. However, among these groups, benzene, furan, thiophene, pyridine, cyclopentane, cyclohexane, pyrrolidine, piperidine, piperazine, morpholine, thiomorpholine, tetrahydropyran (preferably 6-membered ring), and the like are preferred for the "5- to 6-membered ring", inter alia, benzene is preferred.

Examples of the "substituent" optionally possessed by the "5- to 6-membered rings" of the "optionally substituted 5- to 6-membered rings" represented by R¹ include a halogen atom, nitro, cyano, optionally substituted alkyl, optionally substituted cycloalkyl, optionally substituted hydroxyl group, optionally substituted thiol group (wherein the sulfur atom may be oxidized to form an optionally substituted sulfinyl group or optionally substituted sulfonyl group), optionally substituted amino group, optionally substituted acyl group, optionally esterified carboxyl group, and optionally substituted aromatic group.

Examples of the halogen as a substituent for R¹ include a fluorine, chlorine, bromine, and iodine, inter alia, a fluorine and chlorine are preferred.

Examples of the alkyl group in the optionally substituted alkyl as a substituent in R¹ include linear or branched C₁₋₁₀ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₋₆) alkyl. Examples of the substituent in the optionally substituted alkyl include a halogen (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, or the like), and the like, and the number of the substituents is preferably 1 to 3.

Examples of the cycloalkyl group for the optionally substituted cycloalkyl as a substituent for R¹ include a C₃cycloalkyl such as cyclopropyl, cyclobutyl, cyclopentyl, cyclohexyl, cycloheptyl, and the like. Examples of the substituent in the optionally substituted cycloalkyl include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, and the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

Examples of the substituent in the optionally substituted hydroxyl group as a substituent for R¹ include:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₆) alkyl);
(2) an optionally substituted cycloalkyl that may comprise heteroatoms (e.g., C₃₋₇ cycloalkyl such as cyclopropyl, cyclobutyl, cyclopentyl, cyclohexyl and cycloheptyl; saturated 5- to 6-membered heterocyclic group having 1 to 2 heteroatoms such as tetrahydrofuranyl, tetrahydrothienyl, pyrrolidinyl, pyrazolidinyl, piperidyl, piperazinyl, morpholinyl, thiomorpholinyl, tetrahydropyranyl or tetrahydrothiopyranyl (preferably tetrahydropyranyl, etc.); or the like);
(3) an optionally substituted alkenyl (e.g., alkenyl having 2 to 10 carbons such as allyl, crotyl, 2-pentenyl, 3-hexenyl, preferably a lower (C₂₋₆) alkenyl);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted aralkyl (e.g., phenyl-C₁₋₄ alkyl (e.g., benzyl, phenethyl,, etc.) or the like);
(6) a formyl or optionally substituted acyl (e.g., an alkanoyl having 2 to 4 carbons (e.g., acetyl, propionyl, butyryl, isobutyryl, or the like), alkyl sulfonyl having 1 to 4 carbons (e.g., methanesulfonyl, ethanesulfonyl,, etc.) or the like); or
(7) an optionally substituted aryl (e.g., phenyl, naphthyl, etc.), and the like; and examples of the substituents that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl mentioned above include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₆ alkoxy (e.g., methoxy, ethoxy, propoxy, butoxy, trifluoromethoxy, trifluoromethoxy, or the like; preferably an optionally halogenated C₁₋₄ alkoxy), formyl, C₂₋₄ alkanoyl (e.g., acetyl, propionyl, or the like), C₁₋₄ alkyl sulfonyl (e.g., methanesulfonyl, ethanesulfonyl, or the like), optionally substituted 5- to 6-membered aromatic heterocycle {e.g., 5- to 6-membered aromatic heterocyclic ring having 1 to 4 of 1 to 2 kinds of heteroatoms selected from nitrogen atom, sulfur atom and oxygen atom such as furan, thiophene, pyrrole, imidazole, pyrazole, thiazole, oxazole, isothiazole, isoxazole, tetrazole, pyridine, pyrazine, pyrimidine, pyridazine, triazole; and examples of the substituent of the aforementioned heterocyclic ring include a halogen, (e.g., fluorine, chlorine, bromine, iodine, or the like), nitro, cyano, hydroxyl, thiol, amino, carboxyl, optionally halogenated C₁₋₄ alkyl (e.g., trifluoromethyl, methyl, ethyl, or the like), optionally halogenated C₁₋₄ alkoxy (e.g., methoxy, ethoxy, propoxy, butoxy, trifluoromethoxy, trifluoroethoxy, or the like), formyl, C₂₋₄ alkanoyl (e.g., acetyl, propionyl, or the like) or C₁₋₄ alkyl sulfonyl (e.g., methanesulfonyl, ethanesulfonyl, etc.) or the like, and the number of substituents is preferably 1 to 3}, or the like, where the number of substituents is preferably 1 to 3.

The substituent for the optionally substituted thiol group as the substituent of R¹ is exemplified by those for the above "substituents in the optionally substituted hydroxyl group as substituent for R¹," and among these, examples include:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₆) alkyl or the like);
(2) an optionally substituted cycloalkyl (e.g., C₃₋₇ cycloalkyl such as cyclopropyl, cyclobutyl, cyclopentyl, cyclohexyl, cycloheptyl, or the like);
(3) an optionally substituted aralkyl (e.g., phenyl-C₁₋₄ alkyl (e.g., benzyl or phenethyl) or the like); and
(4) an optionally substituted aryl (e.g., phenyl, naphthyl, etc.), and the like, and
   examples of the substituent that may be possessed by the above-mentioned (1) optionally substituted alkyl, (2) optionally substituted cycloalkyl, (3) optionally substituted aralkyl, and (4) optionally substituted aryl include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl or di-C₁₋₄ alkylcarbamoyl, optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of the substituents is preferably 1 to 3.

Examples of the substituent for the optionally substituted amino group as the substituent for R¹ include amino groups having one to two of the same substituents as "substituents in the optionally substituted hydroxyl group as substituent for R¹" above, and among these, preferred examples include
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₆) alkyl or the like);
(2) an optionally substituted cycloalkyl (e.g., C₃₋₇ cycloalkyl such as cyclopropyl, cyclobutyl, cyclopentyl, cyclohexyl, cycloheptyl, or the like);
(3) an optionally substituted alkenyl (e.g., alkenyl having 2 to 10 carbons such as allyl, crotyl, 2-pentenyl and 3-hexenyl, preferably a lower (C₂₋₆) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl and 2-cyclohexenylmethyl, or the like);
(5) a formyl or optionally substituted acyl (e.g., an alkanoyl having 2 to 4 carbons (e.g., acetyl, propionyl, butyryl, or isobutyryl) or alkyl sulfonyl having 1 to 4 carbons (e.g., methanesulfonyl or ethanesulfonyl); and
(6) an optionally substituted aryl (e.g., phenyl, naphthyl, etc.), and the like; and
   examples of the substituent that may be possessed by the above-mentioned (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like) or C₁₋₄ alkyl sulfonyl (e.g., methanesulfonyl, ethanesulfonyl, etc.), and the like, and the number of substituents is preferably 1 to 3.

In addition, with the optionally substituted amino groups as substituents in R¹, two of the substituents on the amino group may be bonded together to form a cyclic amino group (e.g., cyclic amino group having a bond on the nitrogen atom, formed by removing one hydrogen atom from the ring constituent nitrogen atom of a 5- to 6-membered ring such as tetrahydropyrrole, piperazine, piperidine, morpholine, thiomorpholine, pyrrole, imidazole or the like). This cyclic amino group may have substituents, and examples of the substituent include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

Examples of the optionally substituted acyl as a substituent for R¹ include those in which a carbonyl group or sulfonyl group is bonded with the following:
(1) a hydrogen;
(2) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₆) alkyl or the like);
(3) an optionally substituted cycloalkyl (e.g., C₃₋₇ cycloalkyl such as cyclopropyl, cyclobutyl, cyclopentyl, cyclohexyl, cycloheptyl, or the like);
(4) an optionally substituted alkenyl (e.g., alkenyl having 2 to 10 carbons such as allyl, crotyl, 2-pentenyl and 3-hexenyl, and preferably a lower (C₂₋₆) alkenyl or the like);
(5) an optionally substituted cycloalkenyl (e.g., cycloalkenyl having 3 to 7 carbons such as 2-cyclopentenyl, 2-cyclohexyl, 2-cyclopentenylmethyl, 2-cyclohexenylmethyl, or the like);
(6) those wherein an optionally substituted 5- to 6-membered monocyclic aromatic group (e.g., phenyl, pyridyl, etc.) and the like is linked with carbonyl group or sulfonyl group (e.g., formyl, acetyl, propionyl, butyryl, isobutyryl, valeryl, isovaleryl, pivaloyl, hexanoyl, heptanoyl, octanoyl, cyclobutane carbonyl, cyclopentane carbonyl, cyclohexane carbonyl, cycloheptane carbonyl, crotonyl, 2-cyclohexene carbonyl, benzoyl, nicotinoyl, methanesulfonyl, ethanesulfonyl, or the like). Examples of the substituent that may be possessed by the above-mentioned (2) optionally substituted alkyl, (3) optionally substituted cycloalkyl, (4) optionally substituted alkenyl, (5) optionally substituted cycloalkenyl or (6) optionally substituted 5- to 6-membered monocyclic aromatic group include a halogen (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

Examples of the optionally esterified carboxyl group as the substituent in R¹ include those wherein a carbonyloxy group is bonded with
(1) a hydrogen;
(2) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl and decyl, and preferably a lower (C₁₆) alkyl or the like);
(3) an optionally substituted cycloalkyl (e.g., C₃₋₇ cycloalkyl such as cyclopropyl, cyclobutyl, cyclopentyl, cyclohexyl, cycloheptyl, or the like);
(4) an optionally substituted alkenyl (e.g., alkenyl having 2 to 10 carbons such as allyl, crotyl, 2-pentenyl, 3-hexenyl, and preferably a lower (C₂₋₆) alkenyl or the like);
(5) an optionally substituted cycloalkenyl (e.g., cycloalkenyl having 3 to 7 carbons such as 2-cyclopentenyl, 2-cyclohexyl, 2-cyclopentenylethyl, 2-cyclohexenylmethyl, or the like); or
(6) an optionally substituted aryl (e.g., phenyl, naphthyl, etc.), and the like, and preferably a carboxyl, lower (C₁₋₆) alkoxycarbonyl, aryloxycarbonyl (e.g., methoxycarbonyl, ethoxycarbonyl, propoxycarbonyl, phenoxycarbonyl, naphthoxycarbonyl, etc.), and the like. Examples of the substituent that may be possessed by the above (2) optionally substituted alkyl, (3) optionally substituted cycloalkyl, (4) optionally substituted alkenyl, (5) optionally substituted cycloalkenyl, and (6) optionally substituted aryl include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkoxy (e.g., methoxy, ethoxy, propoxy, butoxy, trifluoromethoxy, trifluoroethoxy, or the like), optionally halogenated C₁₋₄ alkoxy-C₁₋₄ alkoxy (e.g., methoxymethoxy, methoxyethoxy, ethoxyethoxy, trifluoromethoxyethoxy, trifluoroethoxyethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

Examples of the aromatic group in the optionally substituted aromatic group as the substituent in R¹ include 5- to 6-membered homocyclic or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl and triazolyl; condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, and the like. Examples of the substituent for these aromatic groups include a halogen (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, propoxy, butoxy, trifluoromethoxy, trifluoroethoxy, or the like), formyl, C₂₋₄ alkanoyl (e.g., acetyl, propionyl, or the like) C₁₋₄ alkyl sulfonyl (e.g., methanesulfonyl, ethanesulfonyl, etc.), and the like, and the number of substituents is preferably 1 to 3.

These substituents in R¹ may be substituted with the same or different 1 to 4 (preferably 1 to 2) at any of positions on the ring. In addition, when the "5- to 6-membered ring" of the "optionally substituted 5- to 6-membered ring" represented by R¹ has two or more substituents, two of these substituents may be bonded together to form a group such as a lower (C₁₋₆) alkylene (e.g., trimethylene, tetramethylene or the like), lower (C₁₋₆) alkyleneoxy (e.g., -CH₂-O-CH₂-, -O-CH₂-CH₂-, -O-CH₂-CH₂-CH₂-, -O-CH₂-CH₂-CH₂-CH₂-, -O-C(CH₃) (CH₃)-CH₂-CH₂- or the like), lower (C₁₋₆) alkylenethio (e.g., -CH₂-S-CH₂-, -S-CH₂-CH₂₋, -S-CH₂-CH₂-CH₂-, -S-CH₂-CH₂-CH₂-CH₂-, -S-C(CH₃)(CH₃)-CH₂-CH₂- or the like), lower (C₁₋₆) alkylenedioxy (e.g., -O-CH₂-O-, -O-CH₂-CH₂-O-, -O-CH₂-CH₂-CH₂-O- or the like), lower (C₁₋₆) alkylenedithio (e.g., -S-CH₂-S-, -S-CH₂-CH₂-S-, -S-CH₂-CH₂-CH₂-S- or the like), oxy lower (C₁₋₆) alkyleneamino (e.g., -O-CH₂-NH-, -O-CH₂-CH₂-NH- or the like), oxy lower (C₁₋₆) alkylenethio (e.g., -O-CH₂-S-, -O-CH₂-CH₂-S- or the like), lower (C₁₋₆) alkyleneamino (e.g., -NH-CH₂-CH₂-, -NH-CH₂-CH₂-CH₂- or the like), lower (C₁₋₆) alkylenediamino (e.g., -NH-CH₂-NH-, -NH-CH₂-CH₂-NH- or the like), thia-lower (C₁₋₆) alkyleneamino (e.g., -S-CH₂-NH-, -S-CH₂-CH₂-NH- or the like), lower (C₂₋₆) alkenylene (e.g., -CH₂-CH=CH-, -CH₂-CH₂-CH=CH-, -CH₂-CH=CH-CH₂ or the like), lower (C₄₋₆) alkadienylene (e.g., -CH=CH-CH=CH-, etc.), and the like.

In addition, the divalent groups that are formed by combining two substituents of R¹ may have 1 to 3 substituents similar to the "substituents" that may be possessed by the "5- to 6-membered ring" of the "optionally substituted 5- to 6-membered ring" (e.g., halogen atom, nitro, cyano, optionally substituted alkyl, optionally substituted cycloalkyl, optionally substituted hydroxyl, optionally substituted thiol group (wherein the sulfur atom may be oxidized, and may form an optionally substituted sulfinyl group or optionally substituted sulfonyl group), optionally substituted amino group, optionally substituted acyl, optionally esterified or amidated carboxyl group, optionally substituted aromatic group or the like).

Specific examples of the "substituent" that the "5- to 6-membered ring" of the "optionally substituted 5- to 6-membered ring" represented by R¹ may have, include a (C₁₋₄) alkyl that may be halogenated or may be alkoxylated with a (C₁₋₄) alkoxy (e.g., methyl, ethyl, t-butyl, trifluoromethyl, methoxymethyl, ethoxymethyl, propoxymethyl, butoxymethyl, methoxyethyl, ethoxyethyl, propoxyethyl, butoxyethyl, or the like); a lower (C₁₋₄) alkoxy that may be halogenated or may be alkoxylated with a (C₁₋₄) alkoxy (e.g., methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, or the like); halogen (e.g., fluorine, chlorine, or the like); nitro; cyano; amino that may be substituted with 1 to 2 of a lower (C₁₋₄) alkyl, formyl, or lower (C₂₋₄) alkanoyl (e.g. an amino, methylamino, dimethylamino, formylamino, acetylamino, or the like); 5-to 6-membered cyclic amino group (e.g., 1-pyrrolidinyl, 1-piperazinyl, 1-piperidinyl, 4-morpholino, 4-thiomorpholino, 1-imidazolyl, 4-tetrahydropyranyl, etc.); and the like.

Examples of the "divalent group wherein the number of atoms constituting the straight chain moiety is 1 to 4" denoted by X¹ and X² include -(CH₂)_{a'}- (where a' denotes an integer of 1 to 4 (with an integer of 1 to 2 being preferred)), -(CH₂)_{b'}-X³- {where b' denotes an integer of 0 to 3 (preferably 0 or 1), and X³ denotes an optionally substituted imino group (e.g., imino group that may be substituted with a lower (C₁₋₆) alkyl, lower (C₃₋₇) cycloalkyl, formyl, lower (C₂₋₇) alkanoyl, lower (C₁₋₆) alkoxycarbonyl, or the like), carbonyl group, oxygen atom, or optionally oxidized sulfur atom (e.g., -S(O)ₘ- (where m denotes an integer of 0 to 2)}, -CH=CH-, -C≡C-, -CO-NH-, - SO₂-NH-, and the like. The bonding of these groups to ring A or ring B can be achieved by either the left or right bond, but with X¹, it is preferable for bonding with ring A to occur via the right-side bond, and with X², it is preferable for bonding with ring B to occur via the left-side bond.

It is preferable for X¹ to be a bond, -(CH₂)_{b'}-O-(wherein b' denotes an integer of 0, 1, or 2 (preferably 0 or 1)), -C≡C- or the like, with a bond being more preferred.

X² is preferably -(CH₂)_{a'}- (where a denotes an integer of 1 to 2), -(CH₂)_{b'}-X³- (where b' denotes an integer of 0 or 1, and X³ denotes an optionally substituted imino group, carbonyl group, oxygen atom, or optionally oxidized sulfur atom), -CH=CH, -CO-NH-, -SO₂-NH- or the like, with -CO-NH-being more preferred.

The divalent group represented by X¹ and X² may have a substituent at any position (preferably on a carbon atom), and examples of the substituent are any substituent that can be bonded to the divalent chain that constitutes the straight chain moiety. Such examples include a lower (C₁₋₆) alkyl (e.g., methyl, ethyl, propyl, isopropyl, butyl, isobutyl, sec-butyl, tert-butyl, pentyl, isopentyl, neopentyl, hexyl, or the like), lower (C₃₋₇) cycloalkyl (e.g., cyclopropyl, cyclobutyl, cyclopentyl, cyclohexyl, cycloheptyl, or the like), formyl, lower (C₂₋₇) alkanoyl (e.g., acetyl, propionyl, butyryl, or the like), optionally esterified phosphono group, optionally esterified carboxyl group, hydroxyl group, oxo, and the like, and preferably a lower alkyl having 1 to 6 carbons (preferably a C₁₋₃ alkyl), hydroxyl, oxo, and the like.

Examples of the optionally esterified phosphono groups include -P(O) (OR⁷) (OR⁸) (wherein the formula, R⁷ or R⁸ each denote a hydrogen, alkyl group having 1 to 6 carbons, or cycloalkyl group having 3 to 7 carbons, and R⁷ and R⁸ may be bonded together to form a 5- to 7-membered ring).

In the above formula, examples of the alkyl group having 1 to 6 carbons represented by R⁷ and R⁸ include methyl, ethyl, propyl, isopropyl, butyl, isobutyl, sec-butyl, tert-butyl, pentyl, isopentyl, neopentyl, hexyl, and the like, and examples of the cycloalkyl having 3 to 7 carbons include cyclopropyl, cyclobutyl, cyclopentyl, cyclohexyl, cycloheptyl, and the like, and a chain lower alkyl having 1 to 6 carbons is preferred, and a lower alkyl hhaving 1 to 3 carbons is more preferred. R⁷ and R⁸ may be the same or different, and are preferably the same. When R⁷ and R⁸ are bonded together to form a 5- to 7-membered ring, R⁷ and R⁸ are bonded together to form a linear C₂₋₄ alkylene side chain represented by -(CH₂)₂-, -(CH₂)₃-, or -(CH₂)₄-. This side chain may have a substituent, and examples of the substituent include a hydroxyl group, halogen, and the like.

Examples of the esterified carboxyl group in the optionally esterified carboxyl group include a group produced by bonding a carboxyl group with an alkyl group having 1 to 6 carbons or a cycloalkyl group having 3 to 7 carbons, examples of which include methoxycarbonyl, ethoxycarbonyl propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, and the like.

Examples of the "5- to 6-membered ring" of the "optionally substituted 5- to 6-membered ring" represented by A in formula (I) above include a 5- to 6-membered saturated or unsaturated alicyclic hydrocarbons such as C₅₋₆ cycloalkane (e.g., cyclopentane, cyclohexane, or the like), C₅₋₆ cycloalkene (e.g., 1-cyclopentene, 2-cyclopentene, 3-cyclopentene, 2-cyclohexene, 3-cyclohexene, or the like), C₅₋₆ cycloalkadiene (e.g., 2,4-cyclopentadiene, 2,4-cyclohexadiene, 2,5-cyclohexadiene, or the like); 6-membered aromatic hydrocarbons such as benzene; 5- to 6-membered aromatic heterocyclic rings, or saturated or unsaturated non-aromatic heterocyclic ring (aliphatic heterocyclic ring), each of which contains at least 1 (preferably 1 to 4, and more preferably 1 or 2) of 1 to 3 kinds (preferably 1 or 2 kinds) of heteroatom selected from an oxygen atom, sulfur atom, nitrogen atom, and the like; and the like.

Herein, examples of the "aromatic heterocyclic ring" include a 5- to 6-membered aromatic monocyclic heterocyclic ring (e.g., furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazan, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, or the like), and examples of the "non-aromatic heterocyclic ring" include 5- to 6-membered saturated or unsaturated non-aromatic heterocyclic rings (aliphatic heterocyclic ring) such as pyrrolidine, tetrahydrofuran, thiolane, piperidine, tetrahydropyran, morpholine, thiomorpholine, piperazine, pyran, oxepine, thiepine, azepine, or the like, or a 5- to 6-membered non-aromatic heterocyclic ring wherein part or all of the double bonds of the above-mentioned aromatic monocyclic heterocyclic ring are saturated or the like.

Examples of the "5- to 6-membered ring" of the "optionally substituted 5- to 6-membered ring" represented by A are preferably 5- to 6-membered aromatic rings, and more preferably benzene, furan, thiophene, pyrrole, pyridine (preferably 6-membered rings), and the like, and particularly preferably benzene.

Examples of the "substituent" that the "5- to 6-membered ring" of the "optionally substituted 5- to 6-membered ring" represented by A may have, include substituents similar to the "substituents" that the "5- to 6-membered ring" of the "optionally substituted 5- to 6-membered ring" represented by R¹ may have. In addition, the substituents for A may be substituted with the same or different 1 to 4 (preferably 1 to 2) at any of positions on the ring, and the substituents may be present at any position if the position is a substitutable position, regardless of whether it is a position represented by E₁ and E₂ or another position.

Examples of the lower alkyl group of the "optionally substituted lower alkyl group" represented by R³ above include C₁₋₆ alkyl such as methyl, ethyl, propyl, isopropyl, butyl, isobutyl, sec-butyl, tert-butyl, pentyl, isopentyl, neopentyl, hexyl, and the like.

Examples of the lower alkoxy groups of the "optionally substituted lower alkoxy group" represented by R³ above include C₁₋₆ alkoxy such as methoxy, ethoxy, propoxy and butoxy.

Examples of the substituent that may be possessed by the "optionally substituted lower alkyl group" and "optionally substituted lower alkoxy group" include a halogen (e.g., fluorine, chlorine, bromine, iodine), hydroxyl group, amino group, mono(lower alkyl) amino, di(lower alkyl)amino, lower alkanoyl, and the like.

Examples of the lower alkyl in the mono(lower alkyl)amino and di(lower alkyl)amino include the same groups as the lower alkyl group of the "optionally substituted lower alkyl group" represented by R³ above.

Examples of the lower alkanoyl include a C₂₋₆ alkanoyl such as acetyl, propionyl, butyryl and isobutyryl.

Examples of the "halogen atom" represented by R³ above include fluorine, chlorine, bromine, iodine, and the like.

Among these groups, an optionally substituted lower C₁₋₆ alkyl group or a halogen atom is preferred for R³, and an optionally substituted methyl group or a halogen atom is particularly preferred.

Examples of the "8- to 10-membered ring" of the "optionally substituted 8- to 10-membered ring" represented by B in formula (I) above include 8- to 10-membered rings optionally having substituents at any substitutable position represented by the formula: 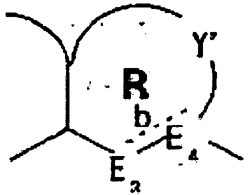 wherein, Y' denotes a divalent group, and the other symbols have the same designations as above.

In the above formula, the divalent group represented by Y' denotes a divalent group whereby ring B forms an optionally substituted 8- to 10-membered ring, and examples include:
(1) -Alkₐ₁-O-Alkₐ₂- (where Alkₐ₁ and Alkₐ₂ each denote a bond or a divalent linear hydrocarbon group having 1 to 5 carbons, provided that the sum of the carbon numbers of Alkₐ₁ and Alkₐ₂ is 5 or less),
(2) -Alk_{b1}-S(O)ₘ-Alk_{b2}- (where m denotes an integer of 0, 1, or 2, Alk_{b1} and Alk_{b2} each denote a bond or a divalent linear hydrocarbon group having 1 to 5 carbons, provided that the sum of the carbon numbers of Alk_{b1} and Alk_{b2} is 5 or less),
(3) -Alk_{d1}- (where Alk_{d1} denotes a divalent linear hydrocarbon group having 4 to 6 carbons),
(4) -Alkₑ₁-NH-Alkₑ₂- (Alkₑ₁ and Alkₑ₂ each denote a bond or a divalent linear hydrocarbon group having 1 to 5 carbons, provided that the sum of the carbon numbers of Alkₑ₁ and Alkₑ₂ is 5 or less), -Alkₑ₆-N=CH-Alkₑ₇-, -Alk,₇-CH=N-Alk,₆-,-Alkₑ₆-N=N-Alkₑ₇- (where Alkₑ₆ and Alkₑ₇ each denote a bond or a divalent linear hydrocarbon group having 1 to 4 carbons, provided that the sum of the carbon numbers of Alkₑ₆ and Alkₑ₇ is 4 or less), and the like.

Examples of these divalent linear hydrocarbon groups include divalent groups such as -CH₂-, -(CH₂)₂-, -(CH₂)₃-,-(CH₂)₄-, -(CH₂)₅-, -(CH₂)₆, -CH=, -CH=CH-, -CH=CH-CH₂-,-CH₂CH=CH-, -CH=CH-CH=CH-, =CH-CH=CH-, -CH₂-CH=CH-CH₂-,-CH=CH-(CH₂)₂-, -CH=CH-(CH₂)₃-, -CH=CH- (CH₂)₄-, and the like.

Specific examples of Y' include -O-(CH₂)₃-, -O-(CH₂)₄-, -O-(CH₂)₅-, -CH₂-O-(CH₂)₂-, -O-CH=CH-CH₂-, S(O)ₘ-(CH₂)₃-(where m denotes an integer of 0 to 2), -S(O)ₘ-(CH₂)₄-(where m denotes an integer of 0 to 2), -S(O)ₘ-(CH₂)₅-(where m denotes an integer of 0 to 2), -CH₂-S(O)ₘ-(CH₂)₂-(where m denotes an integer of 0 to 2), -S(O)ₘ-CH=CH-CH₂-(where m denotes an integer of 0 to 2), -(CH₂)₄-, -(CH₂)₅-, -(CH₂)₆-, -CH=CH-CH=CH-, -CH=CH-(CH₂)₂-, -NH-(CH₂)₃-, -NH-(CH₂)₄-, -NH-(CH₂)₅-, -CH₂-NH-(CH₂)₂-, -NH-CH=CH-CH₂-, -N=CH-CH=CH-, -CH=N-(CH₂)₂-, -CH=N-CH=CH-, -N=N-(CH2)2-, -N=N-CH=CH-, -CH=N-N=CH- (each denoting a bond that starts on ring A), and the like. An 8-membered ring is preferable for ring B.

In addition, the divalent group may have substituents, and examples of the substituent include an oxo group and the same substituents as the "substituents" that the "5- to 6-membered ring" of the "optionally substituted 5- to 6-membered ring" represented by R¹ may have, and among these, a lower (C₁₋₃) alkyl (e.g., methyl, ethyl, propyl, or the like), phenyl, oxo, hydroxyl group, and the like are preferred. The substituents of the divalent group may be the same or different, and 1 to 6 (preferably 1 to 2) of them may be substituted. Any substitution position is acceptable, provided that bonding to the divalent group is possible.

Examples of the "substituent" that the "8- to 10-membered ring" of the "optionally substituted 8- to 10-membered ring" represented by B may have, are an oxo group and the same substituents as the "substituents" that the "5- to 6-membered ring" of the "optionally substituted 5-to 6-membered ring" represented by R¹ may have.

Examples of the divalent group represented by Y are preferably -O-(CH₂)₃-, -O-(CH₂)₄- -O-(CH₂)₅-, -S(O)ₘ-(CH₂)₃-(m denotes an integer of 0 to 2), -S(O)ₘ-(CH₂)₄- (m denotes an integer of 0 to 2), -S(O)ₘ-(CH₂)₅- (m denotes an integer of 0 to 2), -(CH₂)₄-, -(CH₂)₅, -(CH₂)₆-, and a group having a divalent group represented by the formula -N(R⁰)- (wherein, R⁰ denotes a hydrogen atom or a substituent) such as -NH-(CH₂)₃-, -NH-(CH₂)₄- and -NH-(CH₂)₅-, in the main chain. Inter alia, the group having a divalent group represented by the formula -N(R^{o})- (wherein, R^{o} denotes a hydrogen atom or a substituent) in the main chain is preferred.

Preferred examples of R^{o} include a hydrogen atom, optionally substituted hydrocarbon group, optionally substituted heterocyclic group, optionally substituted hydroxyl group, optionally substituted thiol group (wherein the sulfur atom may be oxidized to form an optionally substituted sulfinyl group or optionally substituted sulfonyl group), optionally substituted amino group, optionally esterified or amidated carboxyl group, optionally substituted acyl group, and the like, and more preferably a hydrogen atom, optionally substituted hydrocarbon group, optionally substituted heterocyclic group, optionally substituted acyl group, and the like.

Preferred modes for R⁰ include a hydrogen atom, optionally substituted hydrocarbon group, and optionally substituted acyl group, and as the optionally substituted hydrocarbon group, preferred are an optionally halogenated or hydroxylated C₁₋₆ alkyl and an optionally halogenated or hydroxylated C₂₋₆ alkenyl. Preferred examples of the optionally substituted acyl group include an optionally halogenated or hydroxylated C₁₋₄ alkyl sulfonyl, formyl, optionally halogenated or hydroxylated C₂₋₅ alkanoyl, and the like, and R⁰ is more preferably an optionally halogenated or hydroxylated C₁₋₄ alkyl, a formyl, an optionally halogenated or hydroxylated C₂₋₅ alkanoyl, and the like, inter alia, propyl, isobutyl, isobutenyl, or 3-hydroxy-2-methylpropyl is preferred. Another preferred mode for R⁰ includes groups represented by the formula - (CH₂)ₛ-R^{x} {wherein, s denotes 0 or 1, and R^{x} denotes an optionally substituted 5- to 6-membered monocyclic aromatic group (e.g., the same groups as the "5- to 6-membered monocyclic aromatic groups" exemplified in the paragraph concerning ring A; preferably a phenyl, pyridyl, pyrazolyl, thiazolyl, oxazolyl, tetrazolyl, and the like, each of which may be substituted with a halogen, an optionally halogenated or hydroxylated C₁₋₄ alkyl, optionally halogenated or hydroxylated C₁₋₄ alkoxy, or the like)}.

Examples of the "hydrocarbon group" of the "optionally substituted hydrocarbon group" include:
(1) an alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, sec-butyl, tert-butyl, pentyl, isopentyl, neopentyl, hexyl, heptyl, octyl, nonyl, decyl, preferably a lower (C₁₋₆) alkyl, and more preferably a lower (C₁₋₄) alkyl or the like);
(2) a cycloalkyl (e.g., C₃₋₇ cycloalkyl such as cyclopropyl, cyclobutyl, cyclopentyl, cyclohexyl, cycloheptyl, or the like);
(3) an alkenyl (e.g., alkenyl having 2 to 10 carbons such as allyl, crotyl, 2-pentenyl, 3-hexenyl, and preferably a lower (C₂₋₆) alkenyl or the like);
(4) a cycloalkenyl (e.g., cycloalkenyl having 3 to 7 carbons such as 2-cyclopentenyl, 2-cyclohexenyl, 2-cyclopentenylmethyl, 2-cyclohexenylmethyl);
(5) an alkynyl (e.g., alkynyl having 2 to 10 carbons such as ethynyl, 1-propynyl, 2-propynyl, 1-butynyl, 2-pentynyl, 3-hexynyl, preferably a (C₂₋₆) alkynyl or the like);
(6) an aralkyl (e.g., phenyl-C₁₋₄ alkyl (e.g., benzyl or phenethyl) or the like);
(7) an aryl (e.g., phenyl, naphthyl, or the like);
(8) a cycloalkyl-alkyl (e.g., C₃₋₇ cycloalkyl-C₁₋₄ alkyl such as cyclopropylmethyl, cyclobutylmethyl, cyclopentylmethyl, cyclohexylmethyl, cycloheptylmethyl, etc.); and the like; and the substituents that may be possessed by the above (1) alkyl, (2) cycloalkyl, (3)alkenyl, (4) cycloalkenyl, (5) alkynyl, (6) aralkyl, (7) aryl, and (8) cycloalkyl-alkyl include a halogen (e.g., fluorine, chlorine, bromine, iodine, or the like) nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄-alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, propoxy, butoxy, trifluoromethoxy, trifluoroethoxy, or the like), C₁₋₄ alkylenedioxy (e.g., -O-CH₂-O-, -O-CH₂-CH₂-O-, or the like), optionally substituted sulfonamide (e.g., a group formed by bonding an optionally substituted amino group (e.g., amino, mono C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like) with -SO₂-, or the like), formyl, C₂₋₄ alkanoyl (e.g., acetyl, propionyl, or the like), C₁₋₄ alkyl sulfonyl (e.g., methanesulfonyl, ethanesulfonyl, or the like), optionally substituted heterocyclic group, and the like, and the number of the substituent is preferably 1 to 3.

Examples of the "heterocyclic group" in said "optionally substituted heterocyclic group" and the "optionally substituted heterocyclic group" represented by R⁰ include groups formed by removing one hydrogen atom from aromatic heterocycles or non-aromatic heterocycles. Examples of the aromatic heterocycles include a 5- to 6-membered aromatic heterocycle containing 1 to 4 of one or two kinds of heteroatoms selected from nitrogen atom, sulfur atom and oxygen atom such as furan, thiophene, pyrrole, imidazoles, pyrazole, thiazole, oxazole, isothiazole, isoxazole, tetrazole, pyridine, pyrazine, pyrimidine, pyridazine, triazole, oxadiazole, thiadiazole, and examples of the non-aromatic heterocycle include a 5-to 6-membered non-aromatic heterocycle having 1 to 4 of one or two kinds of heteroatoms selected from nitrogen atom, sulfur atom, and oxygen atom such as tetrahydrofuran, tetrahydrothiophene, dioxolane, dithiolane, oxathiolane, pyrrolidone, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, and a non-aromatic heterocycle in which some or all of the bonds on the aromatic heterocycle are saturated bonds (preferably aromatic heterocycles such as pyrazole, thiazole, oxazole, tetrazole), and the like.

Examples of the "optionally substituted hydroxyl group", "optionally substituted thiol group", "optionally substituted amino group", "optionally esterified carboxyl group", and "optionally substituted acyl group" represented by R⁰ include the same groups as the "optionally substituted hydroxyl group", "optionally substituted thiol group", "optionally substituted amino group", "optionally esterified carboxyl group" and "optionally substituted acyl group" as the substituent that may be possessed by the "5-to 6-membered ring group" of the "optionally substituted 5-to 6-membered ring group" represented by R¹. Examples of the "optionally amidated carboxyl group" include groups wherein the "optionally substituted amino group" is linked with carbonyl group, preferably carbamoyl, mono-C₁₋₆ alkylcarbamoyl, di-C₁₋₆ alkylcarbamoyl, and the like.

The imino group represented by Y^{a} that may have a formyl, optionally substituted C₁₋₆ alkyl, optionally substituted C₂₋₆ alkenyl, optionally substituted aryl, optionally substituted heterocyclic group, optionally substituted aryl-methyl, or optionally substituted heterocyclic-methyl as substituents denotes groups within the definition of the groups described in relation to (R^{o})-represented by Y. Among these groups, it is preferable for R^{o} to be (1) a C₁₋₆ alkyl, (2) a C₂₋₆ alkenyl, (3) a C₆₋₁₀ aryl, (4) a C₆₋₁₀ aryl-methyl, (5) a heterocyclic group, or (6) a heterocyclic-methyl (wherein (1) and (2) may be substituted with halogen or hydroxyl group, and (3), (4), (5), and (6) may be substituted with a halogen, a C₁₋₆ alkyl that may be substituted with a halogen or hydroxyl group, or a C₁₋₆ alkoxy that may be substituted with a halogen or hydroxyl group).

In addition, the substituents of B may be the same or different, and 1 to 7 (preferably 1 to 2) may be substituted at any position (including E₃ and E₄), but it is preferable for the E₃ position to be unsubstituted.

In formula (I) above, compounds are preferred wherein E₃ and E₄ are each an optionally substituted carbon atom (preferably an unsubstituted carbon atom), and b is a double bond.

In formula (I) above, examples of the "divalent cyclic groups" represented by Z¹ are the same groups as the 5- to 6-membered ring of the "optionally substituted 5- to 6-membered ring" represented by R¹, or groups formed by the removal of two hydrogen atoms from a condensed aromatic heterocycle, such as benzofuran, indole, benzothiophene, benzoxazole, benzothiazole, indazole, benzimidazole, quinoline, isoquinoline, quinoxaline, phthalazine, quinazoline, cinnoline, imidazopyridine, and the like. Among these, divalent cyclic groups are preferred which are formed by the removal of two hydrogen atoms from benzene, furan, thiophene, pyridine, pyridazine, pyrimidine, benzimidazole, cyclopentane, cyclohexane, pyrrolidine, piperidine, piperazine, morpholine, thiomorpholine, tetrahydropyran, and the like; and divalent cyclic groups are particularly preferred which are formed by removing 2 hydrogen atoms from benzene, pyridine, pyridazine, benzimidazole, cyclohexane, or piperidine (preferably benzene).

The "divalent cyclic group" represented by Z¹ may have the same substituents as the "substituents" that the "5- to 6-membered ring" of the "optionally substituted 5- to 6-membered ring group" represented by R¹ may have. Among these, preferred substituents include a halogen atom (e.g., fluorine, chlorine, bromine, or the like), C₁₋₄ alkyl group that may be substituted with a halogen atom (e.g., methyl, ethyl, trifluoromethyl, trifluoroethyl, or the like), or C₁₋₄ alkoxy group that may be substituted with a halogen atom (e.g., methoxy, ethoxy, propoxy, trifluoromethoxy, trifluoroethoxy, or the like), but it is preferable not to have substituents except X² and Z². In addition, when Z¹ is a 6-membered divalent cyclic group (preferably phenylene), the substitution position on Z² is preferably the para-position of X². In addition, Z¹ is preferably a phenylene optionally having 1) a halogen atom, 2) a C₁₋₄ alkyl group that may be substituted with a halogen atom, or 3) a C₁₋₄ alkoxy group that may be substituted with a halogen atom, as a substituent, and a phenylene having a methyl group or trifluoromethyl group as a substituent is particularly preferred.

The divalent group represented by Z² in formula (I) above is represented, for example, by the formula -Z^{2a}-W¹-Z^{2b}- (Z^{2a} and Z^{2b} each denote O, S(O)ₘ (wherein m denotes 0, 1, or 2), an optionally substituted imino group (-N(R^{a})-), or a bond, and W¹ denotes an optionally substituted alkylene group, optionally substituted alkenylene group, or a bond). When Z¹ is a benzene ring, for example, the bonding position of Z² may be any position but is preferably the para-position.

Examples of the substituent (R^{a}) of the optionally substituted imino group represented by Z^{2a} and Z^{2b} include a hydrogen atom, optionally substituted lower (C₁₋₆) alkyl {e.g., methyl, ethyl, propyl, isopropyl, butyl, isobutyl, sec-butyl, tert-butyl, pentyl, isopentyl, neopentyl, hexyl, hydroxy-C₁₋₆ alkyl (e.g., hydroxyethyl, hydroxypropyl, hydroxybutyl, or the like), halogenated C₁₋₆ alkyl (e.g., trifluoromethyl, trifluoroethyl, or the like), cyanated C₁₋₆ alkyl (e.g., cyanoethyl, cyanopropyl, or the like), optionally esterified or amidated carboxyl-C₁₋₆ alkyl, and the like}, formyl, lower (C₂₋₅) alkanoyl (e.g., acetyl, propionyl, butyryl, or the like), lower (C₁₋₅) alkyl sulfonyl (methylsulfonyl, ethylsulfonyl, etc.), and the like.

Examples of the alkylene group of the "optionally substituted alkylene group" represented by W¹ include alkylene chains represented by -(CH₂)ₖ₁- (k1 denotes an integer of 1 to 4). Examples of the alkenylene group of the "optionally substituted alkenylene group" represented by W¹ include alkenylene chains represented by -(CH₂)k₂-(CH=CH)-(CH₂)k₃- (wherein k2 and k3 are the same or different and denote 0, 1, or 2, and the sum of k2 and k3 is 2 or less). The alkylene groups and alkenylene groups represented by W¹ may have substituents at any position (preferably on a carbon atom), and any substituent may be present, provided that it is one that can be bonded to the alkylene chain or alkenylene chain that constitutes the linear chain moiety. Examples thereof include a lower (C₁₆) alkyl (e.g., methyl, ethyl, propyl, isopropyl, butyl, isobutyl, sec-butyl, tert-butyl, pentyl, isopentyl, neopentyl, hexyl, or the like), lower (C₃₋₇) cycloalkyl (e.g., cyclopropyl, cyclobutyl, cyclopentyl, cyclohexyl, cycloheptyl, or the like), formyl, lower (C₂₋₇) alkanoyl (e.g., acetyl, propionyl, butyryl, or the like), optionally esterified phosphono group, optionally esterified or amidated carboxyl group, hydroxyl group, oxo, hydroxyimino group, optionally substituted lower (C₁₋₆) alkoxyimino group, and the like, and preferably a lower alkyl having 1 to 6 carbons (preferably a C₁₋₃ alkyl), hydroxyl group, oxo, hydroxyimino group, lower (C₁₋₆) alkoxyimino group (which may be substituted with a polar group such as hydroxyl group, cyano group, optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like)), and the like.

Examples of the optionally esterified phosphono group are those that are represented by P(O) (OR⁹) (OR¹⁰) (wherein, R⁹ and R¹⁰ each denote a hydrogen atom, alkyl group having 1 to 6 carbons, cycloalkyl group having 3 to 7 carbons, or the like; and R⁹ and R¹⁰ can be bonded together to form a 5-to 7-membered ring).

In the above formula, examples of the alkyl group having 1 to 6 carbons represented by R⁹ and R¹⁰ include methyl, ethyl, propyl, isopropyl, butyl, isobutyl, sec-butyl, tert-butyl, pentyl, isopentyl, neopentyl, hexyl, and the like; and examples of the cycloalkyl groups having 3 to 7 carbons include cyclopropyl, cyclobutyl, cyclopentyl, cyclohexyl, cycloheptyl, and the like, and the preferred groups are a chain lower alkyl group having 1 to 6 carbons, and lower alkyl groups having 1 to 3 carbons are more preferred. R⁹ and R¹⁰ may be the same or different, and preferably the same. In addition, when R⁹ and R¹⁰ are bonded together to form a 5- to 7-membered ring, R⁹ and R¹⁰ are bonded together to form a linear C₂₋₄ alkylene side chain represented by -(CH₂)₂-, -(CH₂)₃-, or -(CH₂)₄-. The side chain may have substituents, and examples of such substituents include a hydroxyl group, a halogen, and the like.

Examples of the ester of the optionally esterified carboxyl group include esters formed by bonding carboxyl group with cycloalkyl group having 3 to 7 carbons or an alkyl group having 1 to 6 carbons; for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, cyclopentyloxycarbonyl, cyclohexyloxycarbonyl, and the like.

Examples of the amide of the optionally amidated carboxyl group include those produced by bonding carboxyl group with an alkylamino group having 1 to 6 carbons, cycloalkylamino group having 3 to 7 carbons, or 5- to 8-membered cyclic amine (e.g., pyrrolidine, piperidine, morpholine, or the like); for example, carbamoyl, mono-C₁₋₆ alkylcarbamoyl, di-C₁₋₆ alkylcarbamoyl, cyclopentylaminocarbonyl, cyclohexylaminocarbonyl, pyrrolidinocarbonyl, piperidinocarbonyl, morpholinocarbonyl, thiomorpholinocarbonyl, and the like.

For Z², divalent groups are preferred wherein either one of Z^{2a} and Z^{2b} is O, S(O)ₘ (m is 0, 1, or 2), or -N(R^{a})-(wherein R^{a} denotes a hydrogen atom or an optionally substituted lower C₁₋₄ alkyl group), and the other is a bond, and W is -(CH₂)ₚ- (wherein p denotes an integer of 1 to 3), or Z² is -CH(OH)-. Divalent groups are more preferred wherein either one of Z^{2a} or Z^{2b} is O or S(O)ₘ (m is 0, 1, or 2) and the other is a bond, and W is -(CH₂)ₚ- (where p denotes an integer of 1 to 3) or Z² is -CH(OH)-. Z² is further more preferably -CH₂-, -CH(OH)- or -S(O)ₘ-CH₂-(wherein m denotes 0, 1, or 2), and particularly preferably -S(O)ₘ-CH₂- (m is 0, 1, or 2). When Z^{2a} is bonded to Z¹, - SOCH₂- is particularly preferred.

Z^{2a} denotes a bond, S, SO, or SO₂, among these, SO is preferred, and in such a case, compounds are preferred wherein the steric configuration of the SO is (S).

In the above formula [I], examples of the "optionally substituted amino group wherein the nitrogen atom may be converted to a quaternary ammonium or oxide" represented by R² include an amino group that may have 1 to 2 substituents, and an amino group having three substituents wherein the nitrogen atom has been converted to a quaternary ammonium. When the number of substituents on the nitrogen atom is 2 or more, these substituents may be the same or different, and when the number of substituents on the nitrogen atom is 3, the amino group may be any type of -N⁺R^{p}R^{p}R^{p}, -N⁺R^{P}R^{P}R^{q}, and -N⁺R^{p}R^{q}R^{r} (wherein R^{p}, R^{q}, and R^{r} are each different and denote a hydrogen atom or a substituent). In addition, examples of the counter anion for the amino group wherein the nitrogen atom has been converted to a quaternary ammonium include halogen atom anions (e.g., Cl⁻, Br⁻, I⁻ or the like), as well as anions derived from inorganic acids such as hydrochloric acid, hydrobromic acid, nitric acid, sulfuric acid and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; anions derived from acidic amino acid such as aspartic acid and glutamic acid; and the like, inter alia, Cl⁻, Br⁻, and I⁻ is preferred.

Examples of the substituent of the amino group include:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower (C₁₋₆) alkyl or the like);
(2) an optionally substituted cycloalkyl (e.g., C₃₋₈ cycloalkyl such as cyclopropyl, cyclobutyl, cyclopentyl, cyclohexyl, cycloheptyl, cyanooctyl, or the like);
(2-1) the above cycloalkyl may contain one heteroatom selected from sulfur atom, oxygen atom, and nitrogen atom, and may form an oxirane, thiolane, aziridine, tetrahydrofuran, tetrahydrothiophene, pyrrolidine, tetrahydropyran, tetrahydrothiopyran, tetrahydrothiopyran, 1-oxide, or piperidine (preferably a 6-membered ring such as tetrahydropyran, tetrahydrothiopyran, piperidine, etc.), and the like, and with respect to the bonding site to the amino group, 3- or 4-position (preferably 4-position) is preferred;
(2-2) in addition, the cycloalkyl can condense with a benzene ring to form an indane (e.g., indan-1-yl, indan-2-yl, or the like), tetrahydronaphthalene (e.g., tetrahydronaphthalen-5-yl, tetrahydronaphthalen-6-yl, etc.), and the like (preferably an indane or the like);
(2-3) in addition, the cycloalkyl can crosslink via a linear atom chain having 1 to 2 carbons to form a crosslinked cyclic hydrocarbon residue such as bicyclo[2.2.1]heptyl, bicyclo[2.2.2]octyl, bicyclo[3.2.1]octyl, bicyclo[3.2.2]nonyl, and the like (preferably a cyclohexyl having a cross-link via a linear atomic chain having 1 to 2 carbons, and more preferably bicyclo[2.2.1]heptyl and the like);
(3) an optionally substituted alkenyl (e.g., alkenyl having 2 to 10 carbons such as allyl, crotyl, 2-pentenyl, 3-hexenyl, and preferably a lower (C₂₋₆) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted aralkyl (e.g., phenyl-C₁₋₄ alkyl (e.g., benzyl, phenethyl, or the like);
(6) a formyl or optionally substituted acyl (e.g., an alkanoyl having 2 to 4 carbons (e.g., acetyl, propionyl, butyryl, isobutyryl, or the like), alkyl sulfonyl having 1 to 4 carbons (e.g., methanesulfonyl, ethanesulfonyl, or the like), alkoxycarbonyl having 1 to 4 carbons (e.g., methoxycarbonyl, ethoxycarbonyl, tert-butoxycarbonyl, or the like), or aralkyloxycarbonyl having 7 to 10 carbons (e.g., benzyloxy, carbonyl, etc.), or the like);
(7) an optionally substituted aryl (e.g., phenyl, naphthyl, or the like);
(8) an optionally substituted heterocyclic group (e.g., a group formed by removing one hydrogen atom from a 5- to 6-membered aromatic heterocyclic ring comprising 1 to 4 of 1 or 2 kinds of heteroatoms selected from nitrogen atom, sulfur atom, and oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, and thiadiazole; a group formed by removing one hydrogen atom from a condensed heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, or the like; or a group formed by removing one hydrogen atom from a 5- to 6-membered non-aromatic heterocyclic ring comprising 1 to 4 of one or two kinds of heteroatoms selected from nitrogen atom, sulfur atom, and oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran tetrahydropyran, or the like; preferably a group formed by removing one hydrogen atom from a 5- to 6-membered non-aromatic heterocycle or the like; more preferably a group formed by removing one hydrogen atom from a 5- to 6-membered non-aromatic heterocyclic ring comprising one heteroatom, such as tetrahydrofuran, piperidine, tetrahydropyran, tetrahydrothiopyran, etc.), and the like. In addition, the substituents of the amino group may be bonded together to form a 5- to 7-membered cyclic amino such as piperidine, piperazine, morpholine, and thiomorpholine.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl, and (8) optionally substituted heterocyclic group include a halogen (e.g., fluorine, chlorine, bromine, iodine, or the like), optionally halogenated lower (C₁₋₄) alkyl, lower (C₁₋₄) alkyl that may be substituted with a polar group such as hydroxyl group, cyano group, an optionally esterified or amidated carboxyl group (e.g., hydroxy-C₁₋₄ alkyl, cyano-C₁₋₄ alkyl, carboxyl-C₁₋₄ alkyl, C₁₋₄ alkoxycarbonyl-C₁₋₄ alkyl, carbamoyl-C₁₋₄ alkyl, mono-C₁₋₄ alkylcarbamoyl-C₁₋₄ alkyl, di-C₁₋₄ alkylcarbamoyl, di-C₁₋₄ alkylcarbamoyl-C₁₋₄ alkyl, pyrrolidinocarbonyl-C₁₋₄ alkyl, piperidinocarbonyl-C₁₋₄ alkyl, morpholinocarbonyl-C₁₋₄ alkyl, thiomorpholinocarbonyl-C₁₋₄ alkyl, or the like), optionally halogenated C₁₋₄ alkoxy (e.g., methoxy, ethoxy, propoxy, butoxy, trifluoromethoxy, trifluoroethoxy, or the like), C₁₋₄ alkylenedioxy (e.g., -O-CH₂-O-, -O-CH₂-CH₂-O-, or the like), formyl, C₂₋₄ alkanoyl (e.g., acetyl" propionyl, or the like), C₁₋₄ alkyl sulfonyl (e.g., methanesulfonyl, ethanesulfonyl, or the like), phenyl-lower (C₁₋₄) alkyl, C₃₋₇ cycloalkyl, cyano, nitro, hydroxyl group, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), lower (C₁₋₄) alkoxycarbonyl, lower (C₇₋₁₀) aralkyloxycarbonyl, oxo group (preferably a halogen, optionally halogenated lower (C₁₋₄) alkyl, optionally halogenated lower (C₁₋₄) alkoxy, phenyl-lower (C₁₋₄) alkyl, C₃₋₇ cycloalkyl, cyano, hydroxyl group, etc.), and the like, and the number of substituents is preferably 1 to 3.

The "optionally substituted amino group wherein the nitrogen atom is converted to a quaternary ammonium or an oxide" represented by R² in formula (I) above preferably is an amino group having 1 to 3 substituents selected from:
(1) a linear or branched lower (C₁₋₆) alkyl having 1 to 3 of halogen, cyano, hydroxyl, or C₃₋₇ cycloalkyl;
(2) a C₅₋₈ cycloalkyl which may have 1 to 3 of halogen, optionally halogenated lower (C₁₋₄) alkyl group and phenyl-lower (C₁₋₄) alkyl groups, may contain one heteroatom selected from sulfur atom, oxygen atom and nitrogen atom, may be condensed with a benzene ring and may be crosslinked via a linear atomic chain having 1 to 2 carbons (e.g., cyclopentyl, cyclohexyl, cycloheptyl, cyclooctyl, tetrahydropyranyl, tetrahydrothiopyranyl, piperidinyl, indanyl, tetrahydronaphthalenyl, bicyclo[2.2.1]heptyl, or the like, each of which may be substituted);
(3) a phenyl-lower (C₁₋₄) alkyl that may have 1 to 3 of halogen, optionally halogenated lower (C₁₋₄) alkyl, or optionally halogenated lower (C₁₋₄) alkoxy;
(4) a phenyl that may have 1 to 3 of halogen, optionally halogenated lower (C₁₋₄) alkyl, or optionally halogenated lower (C₁₋₄) alkoxy; and
(5) 5- to 6-membered aromatic heterocyclic group that may have 1 to 3 of halogen, optionally halogenated lower (C₁₋₄) alkyl, optionally halogenated lower (C₁₋₄) alkoxy, optionally halogenated lower (C₁₋₄) alkoxy-lower (C₁₋₄) alkoxy, phenyl-lower (C₁₋₄) alkyl, cyano, and hydroxyl (e.g., a group formed by removing one hydrogen atom from furan, thiophene, pyrrole, pyridine and the like).

The "nitrogen-containing heterocycle" of the "optionally substituted nitrogen-containing heterocyclic group which may comprise sulfur atom or oxygen atom as a ring constituent atom and wherein the nitrogen atom may be converted into a quaternary ammonium or oxide" represented by R² in formula (I) above includes a 5- to 6-membered aromatic heterocycle comprising 1 to 4 of 1 or 2 kinds of heteroatoms selected from nitrogen atom, sulfur atom, and oxygen atom such as pyrrole, imidazole, pyrazole, thiazole, oxazole, isothiazole, isoxazole, tetrazole, pyridine, pyrazine, pyrimidine, pyridazine, triazole, oxadiazole, thiadiazole; a condensed aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine; and a 5- to 8-membered non-aromatic heterocycle that may have 1 to 3 of 1 or 2 kinds of heteroatoms selected from nitrogen atom, sulfur atom and oxygen atom in addition to one nitrogen atom such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azocaine), and the like, and these nitrogen-containing heterocycles may crosslink via a linear atomic chain having 1 to 2 carbons to form crosslinked cyclic nitrogen-containing heterocycles such as azabicyclo[2.2.1]heptane, azabicyclo[2.2.2]octane (quinuclidine), and the like (preferably piperidine having crosslinkage via a linear atomic chain of 1 to 2 carbons).

Among the specific examples of the above nitrogen-containing heterocycle, pyridine, pyridazine, pyrazole, imidazole, triazole, tetrazole, imidazopyridine, pyrrolidine, piperidine, piperazine, morpholine, thiomorpholine and azabicyclo[2.2.2]octane (preferably, pyridine, imidazole, triazole, imidazopyridine, pyrrolidine, piperidine and morpholine).

The nitrogen atom of the "nitrogen-containing heterocycle" may be converted to a quaternary ammonium or may be oxidized. When the nitrogen atom of the "nitrogen-containing heterocycle" is converted to a quaternary ammonium, examples of the counter anion for the "nitrogen-containing heterocyclic group" wherein the nitrogen atom is converted to a quaternary ammonium" include halogen atom anions (e.g., Cl⁻, Br⁻, and I⁻) as well as anions derived from inorganic acid such as hydrochloric acid, hydrobromic acid, nitric acid, sulfuric acid, and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; and anions derived from acidic amino acid such as aspartic acid and glutamic acid, and the like, with Cl⁻, Br⁻, and I⁻ being preferred among them.

The "nitrogen-containing heterocyclic group" may be bonded to the divalent group represented by Z² via either a nitrogen atom or carbon atom, and may be bonded via the ring constituent carbon atom like 2-pyridyl, 3-pyridyl, 2-piperidinyl and the like, and also may be bonded via the ring constituent nitrogen atom, as with: 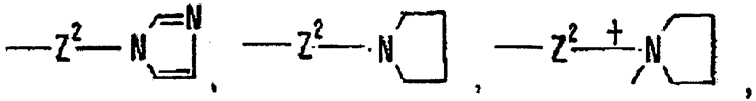 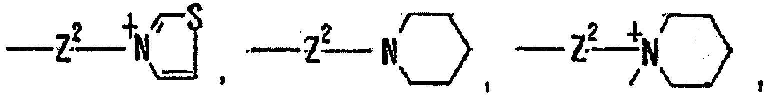 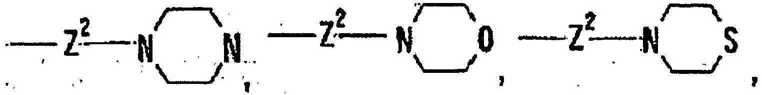 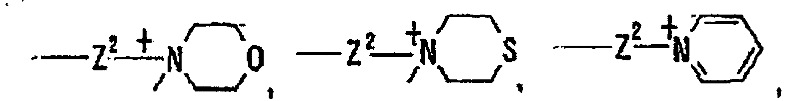 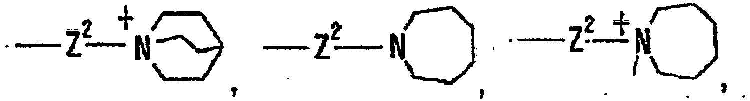 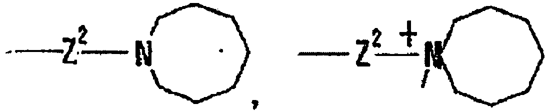 and the like.

Examples of the substituent that the "nitrogen-containing heterocycle" may have, include a halogen, (e.g., fluorine, chlorine, bromine, iodine, or the like), optionally substituted lower (C₁₋₄) alkyl, optionally substituted lower (C₁₋₄) alkoxy, optionally substituted phenyl, optionally substituted mono- or di-phenyl-lower (C₁₋₄) alkyl, optionally substituted C₃₋₇ cycloalkyl, cyano, nitro, hydroxyl, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), lower (C₁₋₄) alkoxycarbonyl, formyl, lower (C₂₋₄) alkanoyl, lower (C₁₋₄) alkyl sulfonyl, optionally substituted heterocyclic group (e.g., a group formed by removing one hydrogen atom from a 5- to 6- membered aromatic heterocyclic ring comprising 1 to 4 of 1 or 2 kinds of heteroatoms selected from nitrogen atom, sulfur atom, and oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, or the like; or a group formed by removing one hydrogen atom from a condensed aromatic heterocyclic group containing 1 to 4 of 1 to 2 kinds of heteroatoms selected from nitrogen atom, sulfur atom and oxygen atom, such as benzofuran, indole, benzothiophene, benzoxazole, benzothiazole, indazole, benzimidazole, quinoline, isoquinoline, quinoxaline, phthalazine, quinazoline, cinnoline, imidazopyridine, or the like; or a group formed by removing one hydrogen atom from a 5- to 6-membered non-aromatic heterocyclic ring comprising 1 to 4 of one or two kinds of heteroatoms selected from nitrogen atom, sulfur atom, and oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, or the like, and the number of the substituents is preferably 1 to 3. In addition, the nitrogen atom of the "nitrogen-containing heterocycle" may be oxidized.

Examples of the substituent that may be possessed by the "optionally substituted lower (C₁₋₄) alkyl", "optionally substituted lower (C₁₋₄) alkoxy", "optionally substituted phenyl", "optionally substituted mono- or di-phenyl lower (C₁₋₄) alkyl", "optionally substituted C₃₋₇ cycloalkyl" and "optionally substituted heterocyclic group" as the substituent that the "nitrogen-containing heterocycle" may have, include a halogen (e.g., fluorine, chlorine, bromine, iodine, or the like), optionally halogenated lower (C₁₋₄) alkyl, lower (C₁₋₄) alkyl that may be substituted with a polar group such as hydroxyl, cyano and an optionally esterified or amidated carboxyl group (e.g., hydroxy-C₁₋₄ alkyl, cyano-C₁₋₄ alkyl, carboxyl-C₁₋₄ alkyl, C₁₋₄ alkoxycarbonyl-C₁₋₄ alkyl, carbamoyl-C₁₋₄ alkyl, mono-C₁₋₄ alkylcarbamoyl-C₁₋₄ alkyl, di-C₁₋₄ alkylcarbamoyl-C₁₋₄ alkyl, pyrrolidinocarbonyl-C₁₋₄ alkyl, piperidinocarbonyl-C₁₋₄ alkyl, morpholinocarbonyl-C₁₋₄ alkyl, thiomorpholinocarbonyl-C₁₋₄ alkyl, or the like), lower (C₃₋₁₀) cycloalkyl, lower (C₃₋₁₀) cycloalkenyl, optionally halogenated C₁₋₄ alkoxy (e.g., methoxy, ethoxy, trifluoromethoxy, trifluoroethoxy, or the like), formyl, C₂₋₄ alkanoyl (e.g., acetyl, propionyl, or the like), C₁₋₄ alkyl sulfonyl (e.g., methanesulfonyl, ethanesulfonyl, or the like), C₁₋₃ alkylenedioxy (e.g., methylenedioxy, ethylenedioxy, or the like), cyano, nitro, hydroxyl group, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), lower (C₁₋₄) alkoxycarbonyl, and the like, and the number of the substituent is preferably 1 to 3.

In the above formula [I], preferred examples of the substituent that may be possessed by the "nitrogen-containing heterocycle" of the "optionally substituted nitrogen-containing heterocyclic group that may comprise sulfur atoms or oxygen atoms as ring constituent atoms and wherein the nitrogen atom may be converted into a quaternary ammonium or oxide" are (1) a halogen, (2) a cyano, (3) a hydroxyl group, (4) a carboxyl group, (5) a carbamoyl group, (6) a lower (C₁₋₄) alkoxycarbonyl, (7) a lower (C₁₋₄) alkylcarbamoyl or 5- to 6-membered cyclic amino (piperidino, morpholino, or the like)-carbonyl, (8) a lower (C₁₋₄) alkyl optionally substituted with a halogen, hydroxyl group, cyano group, lower (C₁₋₄) alkoxy, or optionally esterified or amidated carboxyl group, (9) a lower (C₁₋₄) alkoxy optionally substituted with a halogen, hydroxyl group or lower (C₁₋₄) alkoxy, (10) a phenyl optionally substituted with a halogen, lower (C₁₋₄) alkyl, hydroxyl group, lower (C₁₋₄) alkoxy, or C₁₋₃ alkylenedioxy, (11) a mono- or diphenyl-lower (C₁₋₄) alkyl optionally substituted with a halogen, lower (C₁₋₄) alkyl, hydroxyl group, lower (C₁₋₄) alkoxy, or C₁₋₃ alkylenedioxy, and (12) a group formed by removing one hydrogen atom from a 5- to 6-membered aromatic heterocycle such as furan, thiophene, pyrrole, and pyridine.

In the above formula [I], examples of the "optionally substituted hydrocarbon group" represented by R⁵ and R⁶ in the "group represented by the formula: 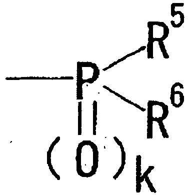 wherein, k denotes 0 or 1, and when k is 0, the phosphorus atom can form a phosphonium salt; R⁵ and R⁶ each denote an optionally substituted hydrocarbon group, optionally substituted hydroxyl group, or optionally substituted amino group (preferably an optionally substituted hydrocarbon group or optionally substituted amino group, more preferably an optionally substituted hydrocarbon group); and R⁵ and R⁶ can be bonded together to form a cyclic group along with an adjacent phosphorus atom)" represented by R² include the following:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower (C₁₋₆) alkyl or the like);
(2) an optionally substituted cycloalkyl (e.g., C₃₋₇ cycloalkyl such as cyclopropyl, cyclobutyl, cyclopentyl, cyclohexyl, cycloheptyl, or the like);
(3) an optionally substituted alkenyl (e.g., alkenyl having 2 to 10 carbons such as allyl, crotyl, 2-pentenyl, 3-hexenyl, and preferably a lower (C₂₋₆) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted alkynyl (e.g., alkynyl having 2 to 10 carbons such as ethynyl, 1-propynyl, 2-propynyl, 1-butynyl, 2-pentynyl, 3-hexynyl, and preferably a lower (C₂₆) alkynyl or the like);
(6) an optionally substituted aralkyl (e.g., phenyl-C₁₋₄ alkyl (e.g., benzyl, phenethyl, etc.), or the like);
(7) an optionally substituted aryl (e.g., phenyl, naphthyl, etc.), and the like, and examples of the substituent that may be possessed by the (1) optionally substituted alkyl, (2) optionally substituted cycloalkyl, (3) optionally substituted alkenyl, (4) optionally substituted cycloalkenyl, (5) optionally substituted alkynyl, (6) optionally substituted aralkyl, and (7) optionally substituted aryl mentioned above include a halogen (e.g., fluorine, chlorine, bromine, iodine, or the like), nitro, cyano, hydroxyl group, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, trifluoromethoxy, trifluoroethoxy, or the like), formyl, C₂₋₄ alkanoyl (e.g., acetyl, propionyl, or the like), and C₁₋₄ alkyl sulfonyl (e.g., methanesulfonyl, ethanesulfonyl, etc.), and the like, and the number of the substituent is preferably 1 to 3.

Examples of the "optionally substituted hydroxyl group" represented by R⁵ and R⁶ include hydroxyl group that may have:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower (C₁₋₆) alkyl or the like);
(2) an optionally substituted cycloalkyl (e.g., C₃₋₇ cycloalkyl such as cyclopropyl, cyclobutyl, cyclopentyl, cyclohexyl, cycloheptyl, or the like),
(3) an optionally substituted alkenyl (e.g., alkenyl having 2 to 10 carbons such as allyl, crotyl, 2-pentenyl, 3-hexenyl, and preferably a lower (C₂₋₆) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) an optionally substituted aralkyl (e.g., phenyl-C₁₋₄ alkyl (e.g., benzyl, phenethyl, etc.), or the like);
(6) a formyl or an optionally substituted acyl (e.g., an alkanoyl having 2 to 4 carbons number (e.g., acetyl, propionyl, butyryl, isobutyryl, or the like), or an alkyl sulfonyl having 1 to 4 carbons (e.g., methanesulfonyl, ethanesulfonyl,, etc.) or the like);
(7) an optionally substituted aryl (e.g., phenyl, naphthyl, etc.), and the like.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, and (7) optionally substituted aryl mentioned above include a halogen (e.g., fluorine, chlorine, bromine, iodine, or the like), nitro, cyano, hydroxyl group, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, trifluoromethoxy, trifluoroethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

In the above formula, R⁵ and R⁶ may be bonded together along with an adjacent phosphorus atom to form a cyclic group (preferably a 5- to 7-membered ring). The cyclic group may have substituents, and examples of such substituents include a halogen, (e.g., fluorine, chlorine, bromine, iodine, or the like), nitro, cyano, hydroxyl, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, or di-C₁₋₄ alkylcarbamoyl), optionally halogenated C₁₋₄ alkyl (e.g., trifluoromethyl, methyl, ethyl, or the like), optionally halogenated C₁₋₄ alkoxy (e.g., methoxy, ethoxy, trifluoromethoxy, trifluoroethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of the substituent is preferably 1 to 3.

Examples of the counter anion when the phosphorus atom forms a phosphonium salt in formula (I) above include halogen atom anions (e.g., Cl⁻, Br⁻, and I⁻) as well as anions derived from inorganic acid such as hydrochloric acid, hydrobromic acid, nitric acid, sulfuric acid and phosphoric acid; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid; anions derived from acidic amino acid such as aspartic acid and glutamic acid; and the like, with Cl⁻, Br⁻, and I⁻ being preferred.

Examples of the optionally substituted amino group represented by R⁵ and R⁶ include amino groups that may have 1 or 2 of:
(1) an optionally substituted alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, and preferably a lower (C₁₋₆) alkyl or the like);
(2) an optionally substituted cycloalkyl (e.g., C₃₋₇ cycloalkyl such as cyclopropyl, cyclobutyl, cyclopentyl, cyclohexyl, cycloheptyl, or the like),
(3) an optionally substituted alkenyl (e.g., alkenyl having 2 to 10 carbons such as allyl, crotyl, 2-pentenyl, 3-hexenyl, and preferably a lower (C₂₋₆) alkenyl or the like);
(4) an optionally substituted cycloalkenyl (e.g., cycloalkenyl having 3 to 7 carbons such as 2-cyclopentenyl, 2-cyclohexenyl, 2-cyclopentenylmethyl, 2-cyclohexenylmethyl, or the like);
(5) formyl or an optionally substituted acyl (e.g., an alkanoyl having 2 to 4 carbons (e.g., acetyl, propionyl, butyryl, isobutyryl, or the like), or an alkyl sulfonyl having 1 to 4 carbons (e.g., methanesulfonyl, ethanesulfonyl, etc.) or the like); and
(6) an optionally substituted aryl (e.g., phenyl, naphthyl, etc.), and the like.

Examples of the substituent that may be possessed by the (1) optionally substituted alkyl, (2) optionally substituted cycloalkyl, (3) optionally substituted alkenyl, (4) optionally substituted cycloalkenyl, (5) optionally substituted acyl, and (6) optionally substituted aryl mentioned above include a halogen (e.g., fluorine, chlorine, bromine, iodine, or the like), nitro, cyano, hydroxyl group, optionally substituted thiol group (e.g., thiol, C₁₋₄ alkylthio, or the like), optionally substituted amino group (e.g., amino, mono-C₁₋₄ alkylamino, di-C₁₋₄ alkylamino, 5- to 6-membered cyclic amino such as tetrahydropyrrole, piperazine, piperidine, morpholine, thiomorpholine, pyrrole, imidazole, or the like), optionally esterified or amidated carboxyl group (e.g., carboxyl, C₁₋₄ alkoxycarbonyl, carbamoyl, mono-C₁₋₄ alkylcarbamoyl, di-C₁₋₄ alkylcarbamoyl, or the like), optionally halogenated C₁₋₄ alkyl (e.g., trifluoromethyl, methyl, ethyl, or the like), optionally halogenated C₁₋₄ alkoxy (e.g., methoxy, ethoxy, trifluoromethoxy, trifluoroethoxy, or the like), formyl, C₂₋₄ alkanoyl (e.g., acetyl, propionyl, or the like), C₁₋₄ alkyl sulfonyl (e.g., methanesulfonyl, ethanesulfonyl, etc.), and the like, and the number of substituents is preferably 1 to 3.

Examples of the substituent in the "optionally substituted amidino group" and "optionally substituted guanidino group" represented by R² are the same as those in the "optionally substituted amino group wherein the nitrogen atom may be converted to a quaternary ammonium or oxide" represented by R² above.

R² is preferably (1) an optionally substituted amino group wherein the nitrogen atom may be converted to a quaternary ammonium or oxide, (2) an optionally substituted nitrogen-containing heterocyclic group which may comprise a sulfur atom or oxygen atom as a ring constituent atom, and wherein the nitrogen atom may be converted to a quaternary ammonium or oxide, (3) an optionally substituted amidino group, or (4) an optionally substituted guanidino group, and R² is more preferably an optionally substituted amino group wherein the nitrogen atom may be converted to a quaternary ammonium or oxide, an optionally substituted nitrogen-containing heterocyclic group which may comprise a sulfur atom or oxygen atom as a ring constituent atom, and wherein the nitrogen atom may be converted to an oxide, and particularly preferred is an optionally substituted amino group or an optionally substituted nitrogen-containing heterocyclic group which may comprise an oxygen atom or sulfur atom as a ring constituent atom.

R² is furthermore preferably a group represented by the formula -NRR" or -N+RR'R" (wherein, R, R', and R" each denote an optionally substituted aliphatic hydrocarbon group (aliphatic chain hydrocarbon group or aliphatic cyclic hydrocarbon group) or an optionally substituted alicyclic (non-aromatic) heterocyclic group), or an optionally substituted nitrogen-containing aromatic heterocyclic group wherein the nitrogen atom may be oxidized.

Examples of the "optionally substituted aliphatic hydrocarbon group" and "optionally substituted alicyclic heterocyclic group" represented by R, R', and R" in the above formula are the same groups as the "optionally substituted aliphatic hydrocarbon groups (e.g., alkyl, cycloalkyl, alkenyl, cycloalkenyl, or the like, each of which may be substituted)" and the "optionally substituted alicyclic heterocyclic groups (e.g., optionally substituted 5- to 6-membered non-aromatic heterocycles and the like)" exemplified for the substituent that the "optionally substituted amino group" represented by substituent R² may have.

Among these groups, optionally substituted chain hydrocarbon groups (e.g., optionally substituted alkyl, alkenyl, and the like) are preferred for R and R', and optionally substituted C₁₋₆ alkyl groups are more preferred, and further an optionally substituted methyl group is particularly preferred.

R" is preferably an optionally substituted alicyclic hydrocarbon group (preferably, an optionally substituted C₃₋₈ cycloalkyl group; more preferably an optionally substituted cyclohexyl) or an optionally substituted alicyclic heterocyclic group (preferably an optionally substituted saturated alicyclic heterocyclic group (preferably a 6-membered cyclic group); more preferably an optionally substituted tetrahydropyranyl, optionally substituted tetrahydrothiopyranyl, or optionally substituted piperidyl; and particularly preferably an optionally substituted tetrahydropyranyl).

In addition, among the pyridine, imidazole, triazole, and imidazopyridine that are exemplified for the preferred "nitrogen-containing aromatic heterocyclic groups" of the "optionally substituted nitrogen-containing aromatic heterocyclic group wherein the nitrogen atom may be oxidized" represented by R², an imidazole or triazole is particularly preferable.

As for the "optionally substituted amino group wherein the nitrogen atom may be converted to a quaternary ammonium or oxide" and the like represented by R²' and R^{2"}, the same as in the corresponding groups of R² mentioned above may be exemplified.

As for the "optionally substituted hydrocarbon group ", "optionally substituted C₁₋₆ alkyl hydrocarbon group" and the like in the substituent represented by R⁴ for the imino group of Y and the substituent for the imino group of Y', the same as in the corresponding groups of R⁰ mentioned above may be exemplified.

The same as in the corresponding groups of W¹ mentioned above may be exemplified for the "optionally substituted alkylene chain" of W².

As the compounds represented by formula (I), the compounds below are preferred.
8-[4-(2-Butoxyethoxy)phenyl]-N-[4-[[N-methyl-N-(tetrahydropyran-4-yl)amino]methyl]phenyl]-3,4-dihydro-2H-1-benzoxocin-5-carboxamide;
8-[4-(2-Butoxyethoxy)phenyl]-N-[4-[[N-methyl-N-(tetrahydropyran-4-yl)amino]methyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfanyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfinyl]phenyl]-1,2,3,4-tetrahydro-1-benzazocine-5-carboxamide;
8-[4-(2-Butoxyethoxy)phenyl]-1-propyl-N-[4-[[[1-propylimidazol-5-yl]methyl]sulfonyl]phenyl]-1,2,3,4-tetrahydro-1-benzazocine-5-carboxamide;
8-[4-(2-Butoxyethoxy)phenyl]-1-isobutyl-N-[4-[[[1-propylimidazol-5-yl]methyl]sulfanyl]phenyl]-1,2,3,4-tetrahydro-1-benzazocine-5-carboxamide;
8-[4-(2-Butoxyethoxy)phenyl]-1-isobutyl-N-[4-[[[1-propylimidazol-5-yl]methyl]sulfinyl]phenyl]-1,2,3,4-tetrahydro-1-benzazocine-5-carboxamide;
8-[4-(2-Butoxyethoxy)phenyl]-1-isobutyl-N-[4-[[[1-propylimidazol-5-yl]methyl]sulfonyl]phenyl]-1,2,3,4-tetrahydro-1-benzazocine-5-carboxamide;
8-[4-(2-Butoxyethoxy)phenyl]-1-isobutyl-N-[4-[[N-methyl-N-(tetrahydropyran-4-yl)amino]methyl]phenyl]-1,2,3,4-tetrahydro-1-benzazocine-5-carboxamide;
(S)-8-[4-(2-Butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-8-[4-(2-Butoxyethoxy)phenyl]-1-propyl-N-[4-[[(1-propyl-1H-imidazol-5-yl)methyl]sulfinyl]phenyl]-1,2,3,4-tetrahydro-1-benzazocine-5-carboxamide methanesulfonate;
(S)-1-isobutyl-8-[4-(2-propoxyethoxy)phenyl]-N-[4-[[(1-propyl-1H-imidazol-5-yl)methyl]sulfinyl]phenyl]-1,2,3,4-tetrahydro-1-benzazocine-5-carboxamide;
(S)-8-[4-(2-Butoxyethoxy)phenyl]-1-[(1-methyl-1H-pyrazol-4-yl)methyl]-N-[4-[[(1-propyl-1H-imidazol-5-yl)methyl]sulfinyl]phenyl]-1,2,3,4-tetrahydro-1-benzazocine-5-carboxamide; and
(S)-8-[4-(2-butoxyethoxy)phenyl]-1-isobutyl-N-[4-[[(4-propyl-4H-1,2,4-triazol-3-yl)methyl]sulfinyl]phenyl]-1,2,3,4-tetrahydro-1-benzazocine-5-carboxamide.

Pharmacologically acceptable salts are preferred for the salts of the compound represented by formula (I), and examples include salts with inorganic bases, salts with organic bases, salts with inorganic acids, salts with organic acids, salts with basic or acidic amino acids, and the like. Suitable examples of the salt with inorganic bases include alkali metal salts such as sodium salts and potassium salts; alkaline earth metal salts such as calcium salts and magnesium salts; aluminum salts and ammonium salts; and the like. Suitable examples of the salt with organic bases include salts with trimethylamine, triethylamine, pyridine, picoline, ethanolamine, diethanolamine, triethanolamine, dicyclohexylamine, N,N'-dibenzylethylenediamine, and the like. Suitable examples of the salt with inorganic acids include salts with hydrochloric acid, hydrobromic acid, nitric acid, sulfuric acid, phosphoric acid, and the like. Suitable examples of the salt with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. Suitable examples of the salt with basic amino acids include salts with arginine, lysine, ornithine, and the like, and suitable examples of the salt with acidic amino acids include salts with aspartic acid, glutamic acid, and the like.

The compounds represented by formula (I) above or salts thereof can be produced according to methods known per se, such as those described in JP-A 2003-335776 and JP-A 08-73476, or analogous methods thereto.

As mentioned above, the pharmaceutical composition of the present invention is a transparent liquid composition that contains a medicinal compound, an oily base, a surfactant and a polar solvent that is poor solvent for the medicinal compound, and wherein the content of the medicinal compound exceeds the solubility thereof in the mixture of the oily base and surfactant.

The composition of the present invention can be manufactured by a method known per se. That is, the components other than the medical active component and the polar solvent are heated with a hot-water bath or other method to give a mixed solution wherein each component is dissolved. Then, the medical active component is added to the mixed solution, which is mixed thoroughly to give a uniform dispersion of the medicinal compound. Polar solvent is then added thereto, and stirring is continued under heating to produce a transparent pharmaceutical composition. In addition, the pharmaceutical composition can be filled into a capsule according to a conventional method.

By enclosing the pharmaceutical composition of the present invention, new preparations for oral use can be prepared, and examples of such preparations include a soft capsule, hard capsule, stick pack, drink, or liquid to be weighed out at the time of use. These preparations can be manufactured by the methods described in the general preparation principles of the 14^{th} revised version of Japanese Pharmacopoeia.

When administered orally the composition of the present invention containing a medicinal compound, in particular, a hardly water-soluble or water-insoluble medicinal compound in an elevated amount, or the preparation of the present invention enclosing the composition, a stable microemulsion in which fine particles comprising the medicinal compound are dispersed, is formed or maintained in the digestive tract. Therefore, the absorbability of the medicinal compound, in particular, a hardly water-soluble or water-insoluble medicinal compound from the digestive tract has dramatically improved, and thereby its bioavailability is increased.

Since the salt of the compound represented by formula (I) above has a superior CCR antagonistic action, in particular, CCR5 and/or CCR2 antagonistic action, inter alia, potent CCR5 antagonistic action, it can be used for the prevention or treatment of HIV infection in human, for example, AIDS, and the prevention or treatment of various other diseases. In addition, the salt of the compound represented by formula (I) above is low toxic and can be safely used.

For example, the pharmaceutical composition comprising the salt of the compound represented by formula (I) above can be used as a CCR5 antagonist, for example, a preventive or therapeutic agent for AIDS and an inhibitor for the progression of pathology of AIDS. Moreover, the pharmaceutical composition comprising the salt of the compound represented by formula (I) above can be used as a prophylactic or therapeutic agent for various diseases such as a prophylactic or therapeutic agent for transplant graft-versus-host disease and/or rejection reactions, and a prophylactic or therapeutic agent for chronic rheumatoid arthritis, autoimmune disease, allergic diseases, ischemic brain cell injury, myocardial infarct, chronic nephritis, and arterial sclerosis.

Examples of the object disease for the preventive or therapeutic agent of the present invention include transplant rejection reactions (post-transplant rejection reactions, post-transplant erythrocytosis/hypertension/organ injury/vascular thickening, graft-versus-host reaction, and the like), rigid myelitis and other arthritic bone diseases (chronic rheumatoid arthritis, arthritis deformans, rheumatoid myelitis, osteoporosis, cellular or other hyperplasia, bone fracture, bone refracture, osteomalasia, bone Piaget's disease, osteomyelitis, osteoarthritis of the knee, joint tissue destruction in similar diseases, and the like), autoimmune diseases (collagenosis, systemic erythematodes, pachydermia, polyarteritis nodosa, myasthenia gravis, multiple sclerosis, and the like), allergic diseases (allergic rhinitis, conjunctivitis, digestive tract allergies, pollinosis, anaphylaxy, atopic dermatitis, bronchial asthma, and the like), inflammatory bowel diseases (ulcerative colitis, Crohn's disease, gastritis, gastric ulcer, stomach cancer, postoperative stomach injury, indigestion, esophageal ulcer, pancreatitis, colonic polyp, gallstones, hemorrhoids, digestive illnesses, localized ileitis, and the like), inflammatory diseases (retinopathy, inflammation subsequent to surgery or injury, relief of swelling, pharyngitis, cystitis, meningitis, inflammatory eye diseases, and the like), respiratory diseases (common cold, pneumonia, asthma, pulmonary hypertension, pulmonary thrombus, pulmonary embolism, pulmonary sarcoidosis, pulmonary tuberculosis, interstitial pneumonitis, pulmonary silicosis, adult respiratory distress syndrome, chronic obstructive pulmonary disease, and the like), infectious diseases (viral infections from cytomegalovirus, influenza virus, herpes virus, and the like, as well as rickettsial infections, bacterial infections, sexually transmitted diseases, Pneumocystis carinii pneumonia, Helicobacter pylori infection, systemic fungal infection, tuberculosis, aggressive Staphylococcus infection, critical viral encephalitis, acute bacterial meningitis, AIDS encephalitis, toxemia, sepsis, critical sepsis, toxemic shock, endotoxic shock, toxic shock syndrome, and the like), cancer and accompanying cachexia, cancer metastasis (urinary bladder cancer, breast cancer, cervical cancer, ovarian cancer, chronic lymphatic leukemia, chronic myeloid leukemia, colon cancer, rectal cancer, colonic cancer, multiple myeloma, acute myeloma, prostate cancer, lung cancer, stomach cancer, Hodgkin's disease, acute melanoma, acute lymphoma, and the like), non-Hodgkin's lymphoma, non-small cell lung cancer, acute melanoma, degenerative neurological diseases (Alzheimer's disease, Parkinson's disease, amyotrophic lateral sclerosis (ALS), Huntington's disease, diabetic neurological impairment, Creutzfeldt-Jacob disease, and the like), neurological diseases (depression, epilepsy, alcohol dependency, and the like), schizophrenia, arterial function insufficiency, central nervous impairment (symptoms and complications resulting from cerebral hemorrhage or cerebral infarct, external head injury, spinal cord injury, cerebral edema, cognitive function impairment, cognitive function abnormalities, autonomic nervous function impairment, autonomic nervous function abnormality, and the like), central nervous injury (external head injury, spinal cord injury, whiplash, and the like), vascular dementia (multiple infarct dementia, Binswanger's disease, and the like), cerebrovascular damage (asymptomatic cerebrovascular damage, transient cerebral ischemic attack, apoplexy, cerebral vascular dementia, hypertensive encephalopathy, and the like), recurrence of cerebral vascular damage and attendant diseases (neurologic symptoms, psychological symptoms, subjective symptoms, impairment of daily activity, and the like), cerebrovascular dementia, post-cerebrovascular-infarct central nervous impairment, cerebrocirculatory injury or abnormality, loss of renal circulation self-regulatory capacity, blood-brain barrier injury, anxiety, unstable angina pectoris and other acute coronary arteriopathic syndromes, mental malaise, amnesia, trigeminal neuralgia, ear, nose, and throat diseases (Meniere's syndrome, tinnitis, dysgeusia, vertigo, disorder of balance, difficulty swallowing, and the like), migraine, chronic pain, skin disorder (keloid, vascular edema, psoriasis, and the like), occlusive arteriosclerosis, occlusive thromboangitis, peripheral arterial occlusion, post-ischemic reperfusion injury, Raynaud's syndrome, Buerger's syndrome, myocarditis, myocardial ischemia, myocardial infarct, post-myocardial-infarct progressive cardiac insufficiency, myocardosis, cardiomegaly, chronic cardiac insufficiency including acute cardiac insufficiency and stasis, stenocardia, arrhythmia, tachycardia, abnormal diurnal blood pressure fluctuation, blood or corpuscular component abnormalities (platelet hypercoagulation, abnormal erythrocyte plasticity, leukocyte adhesion stimulation, blood hyperviscosity, erythrocytosis, vascular purpura, autoimmune hemolytic anemia, disseminated intravascular coagulation syndrome, multiple myeloma, and the like), arterial sclerosis including atheroma (aneurysm, coronary arteriosclerosis, cerebral arteriosclerosis, peripheral arterial sclerosis, and the like), post-bypass vascular reocclusion or restenosis, post-intervention (transdermal coronary arterioplasty, stent placement, coronary arterial endoscopy, vascular ultrasound, coronary perfusion thrombolysis, and the like), vascular stenosis, occlusion and organ injury, generation or hyperfunction of vasoactive substances or blood clotting substances (endoserine, thromboxane A2, and the like), neovascularization (including abnormal vasculature formation in capillary network dystrophy at the outer membrane of arteriosclerotic lesions), thrombosis, fatty deposition stimulation, eye diseases (glaucoma, ocular hypertension, and the like), hypertension, hypertensive tinnitis, dialysis hypotension, endothelial cell and organ injury, endocrine diseases (Addison's disease, Cushing's syndrome, melanocytoma, primary hyperaldosteronism, nephritis, kidney diseases (nephritis, glomerulonephritis, glomerulosclerosis, renal insufficiency, thrombotic microangiopathy, diabetic neuropathy, and the like), glucose tolerance abnormalities, liver disease (hepatitis including chronic hepatitis, cirrhosis of the liver, and the like), interstitial hepatopathy, chronic pancreatitis, portal hypertension, obesity, male infertility, gynecological diseases (climacteric disorder, gestational toxicosis, endometriosis, hysteromyoma, fibroid, ovary disease, breast disease, and the like), breast tumor, chronic fatigue syndrome, prostatomegaly, Behcet's disease, Hodgkin's disease, lacunar infarct, consciousness disorder, psoriasis, diseases resulting from environmental or occupational factors (radiation injury, ultraviolet/infrared/laser light injury, mountain sickness, and the like), and claudicatio intermittens.

The dosage of the composition comprising the salt of the compound represented by formula (I) above of the present invention can be selected appropriately depending on the administration subject, the age and body weight of the administration subject, symptoms, administration time, administration method, and dosage form.

The dose to specific patients is to be determined in consideration of age, body weight, general physical condition, sex, food, administration time, administration method, excretion rate, and extent of the disease at the time of patient treatment, as well as other factors.

When the above composition is to be used as a prophylactic or therapeutic agent for AIDS and inhibitor for progression of pathology of AIDS, the dosage differs depending on the patient condition, body weight, and administration method, and for oral administration, it is about 5 to 1000 mg, preferably about 10 to 600 mg, more preferably about 10 to 300 mg, and particularly preferably about 15 to 150 mg in terms of active ingredient (compound represented by formula (I)) per adult (body weight 50 kg), and it is administered in a single dose or in 2 to 3 divided doses per day.

When the composition comprising the salt of the compound represented by formula (I) above is to be used as a prophylactic or therapeutic agent for graft-versus-host disease and/or rejection reaction in cases of organ transplantation such as the heart, kidney, liver, and bone marrow, it is administered from three days before transplantation, and continuously administered after transplantation. The daily dosage of the pharmaceutical composition of the present invention will differ depending on the patient condition, body weight, and administration method, and for oral administration, it is about 5 to 1000 mg, preferably about 10 to 600 mg, more preferably about 10 to 300 mg, and particularly preferably about 15 to 150 mg in terms of active ingredient (compound represented by formula (I)) per adult (body weight 50 kg), and it is administered in a single dose or in 2 to 3 divided doses per day. In addition, in this case, the composition may be used in combination with other inhibitors for graft-versus-host disease and/or rejection reaction at the time of organ transplantation. Specific examples of the inhibitors for graft-versus-host disease and/or rejection reaction used in combination with the compound represented by formula (I) above or a salt thereof include cyclosporine, tacrolimus, rapamycin, steroids, azathioprine, mycophenolate mofetil, mizoribine, and the like. When these drugs are used in combination, if one of the drugs has an influence on the metabolism of another drug, then the dosages of the respective drugs are to be adjusted appropriately, but in general, the dosage in the single administration of each drug is used.

When the salt of the compound represented by formula (I) above is used for object diseases other than inhibitors for graft-versus-host disease and/or rejection reaction in cases of organ transplantation, the daily dosage will vary depending on the kind of disease, the patient condition and body weight, and the administration method, but for oral administration, it is about 5 to 1000 mg, preferably about 10 to 600 mg, more preferably about 10 to 300 mg, and particularly preferably about 15 to 150 mg, in terms of active ingredient (compound represented by formula (I)) per adult (body weight 50 kg), and it is administered in a single dose or in 2 to 3 divided doses per day. In addition, when used in combination with other drugs, the dosage of the other drugs is to be selected appropriately within, for example, the range of from about 1/200 to 1/2 or more, to about 2 to 3 times or less of the normal dosage. In addition, when 2 or more drugs are used in combination, if one of the drugs has an influence on the metabolism of another drug, then the dosages of the respective drugs are to be adjusted appropriately, but in general, the dosage in the single administration of each drug is used.

In addition, the salt of the compound represented by formula (I) above can be contained in, or used in combination with, blood for transfusion or a blood preparation. Although blood for transfusion or a blood preparation is normally manufactured by mixing blood taken from multiple individuals, there is a case where cells that are not infected and cells that are infected with HIV virus are mixed, and in this case, there is a danger of infection in cells that have not been infected. By blending the compound represented by formula (I) of the present invention, it is possible to prevent or inhibit these viral infection and propagation. In particular, when storing a blood preparation, blending the compound represented by formula (I) is effective for preventing or inhibiting viral infection and propagation. In addition, when blood for transfusion or a blood preparation in which HIV virus is admixed has been administered, by blending the compound represented by formula (I) therein, it is possible to prevent HIV infection and propagation in the individual who was administered the blood for transfusion or blood preparation. For example, when administered orally to adults (body weight about 60 kg) in order to prevent HIV infection during transfusion or during use of a blood preparation, the single dose is normally about 0.02 to 50 mg/kg, preferably 0.05 to 30 mg/kg, and more preferably about 0.1 to 10 mg/kg in terms of CCR antagonist, and it is preferably administered from about 1 to 3 times per day. Of course, the dosage range may be adjusted based on a unit required to divide the daily dose, but as stated above, the dose is determined in consideration of the properties and extent of the disease; the age, body weight, general physical condition, and sex of the patient; food; administration time; administration method; excretion rate; and other factors. The administration method may also be selected appropriately in this case, and the above HIV infection preventive agent of the present invention may be added directly to blood for transfusion or a blood preparation prior to transfusion or prior to the use of the blood preparation. In such case, it is desirable to mix the agent immediately before to 24 hrs before, preferably immediately before to 12 hrs before, and more preferably immediately before to 6 hrs before the transfusion or use of the blood preparation.

When the preventive agent for HIV infection of the present invention is to be administered separately from the blood to be transfused or a blood preparation at the time of transfusion or use of the blood preparation, it is preferable to administer 1 hr before the transfusion or use of the blood preparation to simultaneously, and it is more preferable to continue the administration of 1 to 3 times per day for 4 weeks.

In addition, when the salt of the compound represented by formula (I) is used in combination with a reverse transcriptase inhibitor and/or protease inhibitor, the dosage of the reverse transcriptase inhibitor or protease inhibitor, for example, is selected appropriately with a range of from about 1/200 to 1/2 or more to about 2 to 3 times or less relative to the ordinary dosage.

Examples of ordinary dosage for typical reverse transcriptase inhibitors and protease inhibitors are shown below.
Zidovudine: 100 mg
Didanosine: 125-200 mg
Zalcitabine: 0.75 mg
Lamivudine: 150 mg
Stavudine: 30-40 mg
Saquinavir: 600 mg
Ritonavir: 600 mg
Indinavir: 800 mg
Nelfinavir: 750 mg

In addition, specific embodiments are shown below in which the salt of the compound represented by formula (I) is used in combination with a reverse transcriptase inhibitor and/or protease inhibitor.
(a) About 10 to 300 mg of the compound represented by formula (I) or a salt thereof per an adult (body weight 50 kg) is administered in a form of combined use with about 50 to 200 mg of zidovudine to the same subject. Each of the drugs may be administered simultaneously, or may be administered at different times within a 12-hour period.
(b) About 10 to 300 mg of the compound represented by formula (I) or a salt thereof per an adult (body weight 50 kg) is administered in a form of combined use with about 300 to 1200 mg of saquinavir to the same subject. Each of the drugs may be administered simultaneously, or may be administered at different times within a 12-hour period.

Hereinafter, the present invention will be described further in detail based on Examples, Reference Examples, and Test Examples, but the present invention is not restricted to these Examples.

### Example 1

1 g of (S)-(-)-8-[4-(2-butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate (compound A) was dispersed under heating at 60°C in 6.8 g of polyethylene glycol(8)-caprylic acid/capric acid glycerides and 1.7 g of medium-chain fatty acid triglycerides. Then, 0.5 g of purified water was added to this dispersion liquid, and warmed to obtain a transparent composition solution. The theoretical component ratios are shown in Table 1.

**[Table 1]**

| Component ratio | (-) |
|---|---|
| Compound A | 1.0 |
| Polyethylene glycol(8)-caprylic acid/capric acid glycerides | 6.8 |
| Medium-chain fatty acid triglycerides | 1.7 |
| Purified water | 0.5 |

### Example 2

1 g of compound A was dispersed under heating at 60°C in 3.4 g of polyoxyethylene(40)-hydrogenated castor oil, 3.4 g of polyethylene glycol(8)-caprylic acid/capric acid glycerides, and 1.7 g of medium-chain fatty acid triglycerides. Then, 0.5 g of purified water was added to this dispersion liquid, and warmed to obtain a transparent composition solution. The theoretical component ratios are shown in Table 2.

**[Table 2]**

| Component ratio | (-) |
|---|---|
| Compound A | 1.0 |
| Polyoxyethylene(40)-hydrogenated castor oil | 3.4 |
| Polyethylene glycol(8)-caprylic acid/capric acid glycerides | 3.4 |
| Medium-chain fatty acid triglycerides | 1.7 |
| Purified water | 0.5 |

### Example 3

1 g of compound A was dispersed under heating at 60°C in 3.4 g of polyoxyethylene(60)-hydrogenated castor oil, 3.4 g of polyethylene glycol(8)-caprylic acid/capric acid glycerides, and 1.7 g of medium-chain fatty acid triglycerides. Then, 0.5 g of purified water was added to this dispersion liquid, and warmed to obtain a transparent composition solution. The theoretical component ratios are shown in Table 3.

**[Table 3]**

| Component ratio | (-) |
|---|---|
| Compound A | 1.0 |
| Polyoxyethylene(60)-hydrogenated castor oil | 3.4 |
| Polyethylene glycol(8)-caprylic acid/capric acid glycerides | 3.4 |
| Medium-chain fatty acid triglycerides | 1.7 |
| Purified water | 0.5 |

### Example 4

2 g of compound A was dispersed under heating at 60°C in 3.2 g of polyoxyethylene(40)-hydrogenated castor oil, 3.3 g of polyethylene glycol(8)-caprylic acid/capric acid glycerides, and 1.0 g of medium-chain fatty acid triglycerides. Then, 1.0 g of purified water was added to this dispersion liquid, and warmed to obtain a transparent composition solution. The theoretical component ratios are shown in Table 4.

**[Table 4]**

| Component ratio | (-) |
|---|---|
| Compound A | 2.0 |
| Polyoxyethylene(40)-hydrogenated castor oil | 3.2 |
| Polyethylene glycol(8)-caprylic acid/capric acid glycerides | 3.3 |
| Medium-chain fatty acid triglycerides | 1.0 |
| Purified water | 1.0 |

### Example 5

1 g of compound A was dispersed under heating at 60°C in 2.2 g of lauroyl macrogol(32) glycerides, 4.6 g of polyethylene glycol(8)-caprylic acid/capric acid glycerides, and 1.7 g of medium-chain fatty acid triglycerides. Then, 0.5 g of purified water was added to this dispersion liquid, and warmed to obtain a transparent composition solution. The theoretical component ratios are shown in Table 5.

**[Table 5]**

| Component ratio | (-) |
|---|---|
| Compound A | 1.0 |
| Lauroyl macrogol(32) glycerides | 2.2 |
| polyethylene glycol(8)-caprylic acid/capric acid glycerides | 4.6 |
| caprylic acid/capric acid triglycerides | 1.7 |
| Purified water | 0.5 |

### Example 6

500 g of compound A was dispersed under heating at 60°C in 1700 g of polyoxyethylene(40)-hydrogenated castor oil, 1700 g of polyethylene glycol(8)-caprylic acid/capric acid glycerides, and 850 g of medium-chain fatty acid triglycerides. Then, 250 g of purified water was added to the dispersion liquid, and warmed to obtain a transparent composition solution. About 4600 of soft gelatin capsules were produced wherein 293 mg of the resulting transparent composition solution was enclosed per capsule. The theoretical composition per capsule is shown in Table 6.

**[Table 6]**

| Composition | (mg) |
|---|---|
| Compound A | 30 |
| polyoxyethylene(40)-hydrogenated castor oil | 102 |
| polyethylene glycol(8)-caprylic acid/capric acid glycerides | 102 |
| Medium-chain fatty acid triglycerides | 51 |
| Purified water | 15 |
| Subtotal | 300 |
| Empty soft capsule (transparent) | 180 |
| Total | 480 |

### Example 7

450 g of compound A was dispersed under heating at 60°C, in 1530 g of polyoxyethylene(40)-hydrogenated castor oil, 1530 g of polyethylene glycol(8)-caprylic acid/capric acid glycerides, and 765 g of medium-chain fatty acid triglycerides. Then, 225 g of purified water was added to this dispersion liquid, and warmed to obtain a transparent composition solution. About 10,000 of hard gelatin capsules were produced wherein 284 mg of the resulting transparent composition solution was enclosed per capsule. The theoretical composition per capsule is shown in Table 7.

**[Table 7]**

| Composition | (mg) |
|---|---|
| Compound A | 28.4 |
| Polyoxyethylene(40)-hydrogenated castor oil | 96.56 |
| Polyethylene glycol(8)-caprylic acid/capric acid glycerides | 96.56 |
| Medium-chain fatty acid triglycerides | 48.28 |
| Purified water | 14.2 |
| Subtotal | 284 |
| Empty hard capsule (transparent) | 60 |
| Total | 344 |

### Example 8

1 g of compound A was dispersed under heating at 60°C in 6.8 g of propylene glycol monocaprylate and 1.7 g of medium-chain fatty acid triglycerides. Then, 0.5 g of purified water was added to this dispersion solution, and warmed to obtain a transparent composition solution. The theoretical component ratios are shown in Table 8.

**[Table 8]**

| Component ratio | (-) |
|---|---|
| Compound A | 1.0 |
| Propylene glycol monocaprylate | 6.8 |
| Medium-chain fatty acid triglycerides | 1.7 |
| Purified water | 0.5 |

### Reference Example 1

1 g of compound A was dispersed under heating at 60°C in 3.4 g of polyoxyethylene(40)-hydrogenated castor oil, 3.4 g of polyethylene glycol(8) caprylic acid/capric acid glycerides, and 2.2 g of medium-chain fatty acid triglycerides. A transparent composition was not obtained with this composition. The theoretical component ratios are shown in Table 9.

**[Table 9]**

| Component ratio | (-) |
|---|---|
| Compound A | 1.0 |
| Polyoxyethylene(40)-hydrogenated castor oil | 3.4 |
| Polyethylene glycol(8)-caprylic acid/capric acid glycerides | 3.4 |
| Medium-chain fatty acid triglycerides | 2.2 |

### Evaluation Example 1

When compared the external appearances of the capsules wherein the compositions obtained in Example 7 and Reference example 1 were filled into empty hard gelatin capsules (transparent; charge amount 284 mg), as is clear from Figure 1, a transparent solubilized capsule was obtained with the composition of Example 7, while the medicinal compound was not sufficiently dissolved and resulted in a suspension with the composition obtained in Reference example 1.

### Industrial Applicability

The pharmaceutical composition of the present invention contains a medicinal compound, in particular, a hardly water-soluble or water-insoluble medicinal compound in an elevated amount, and when administered orally, a stable microemulsion is formed in the digestive tract wherein fine particles containing the medicinal compound are dispersed, and thus, there are provided a composition for oral use which is excellent in the absorbability of the medicinal compound via the digestive tract and has a high bioavailability thereof, and a preparation for oral use enclosing the composition.

## Claims

1. A liquid transparent pharmaceutical composition comprising
(i) a polar solvent in an amount of 1 w/w% to 20 w/w%, wherein the polar solvent is water,
(ii) an oily base,
(iii) a surfactant having an HLB of 14 or more, wherein the content (w/w%) of the surfactant is more than the content (w/w%) of the oily base,
(iv) a medicinal compound having a solubility of less than 0.1 mg/ml in water at 25°C, wherein the content of the medicinal compound in the composition is 1 w/w% or more and wherein the content of the medicinal compound is an amount exceeding the solubility thereof in the mixture of the oily base and surfactant, wherein the medicinal compound is a salt of the compound represented by formula (I): 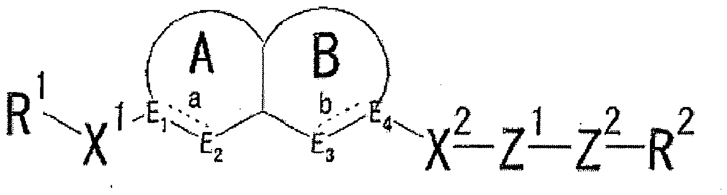
wherein, R¹ denotes an optionally substituted 5- to 6-membered ring,
X¹ denotes a bond or a divalent group wherein the number of atoms constituting the straight-chain moiety is 1 to 4,
ring A denotes an optionally substituted 5- or 6-membered ring, and ring B denotes an optionally substituted 8- to 10-membered ring,
E₁ and E₄ each denote an optionally substituted carbon atom or an optionally substituted nitrogen atom,
E₂ and E₃ each denote an optionally substituted carbon atom, optionally substituted nitrogen atom, optionally oxidized sulfur atom or oxygen atom,
a and b each denote a single bond or a double bond,
X² denotes a divalent group wherein the number of atoms constituting the straight chain moiety is 1 to 4,
Z¹ denotes a bond or a divalent cyclic group,
Z² denotes a bond or a divalent group,
R² denotes (1) an optionally substituted amino group wherein the nitrogen atom may be converted into a quaternary ammonium or oxide, (2) an optionally substituted nitrogen-containing heterocyclic group that may comprise sulfur atoms or oxygen atoms as ring constituent atoms, wherein the nitrogen atom may be converted into a quaternary ammonium or oxide, (3) a group represented by the formula: 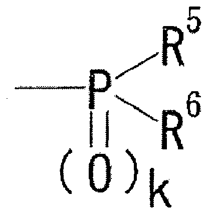
wherein, k denotes 0 or 1, and when k is 0, the phosphorus atom can form a phosphonium salt, R⁵ and R⁶ each denote an optionally substituted hydrocarbon group, optionally substituted hydroxyl group, or optionally substituted amino group, and R⁵ and R⁶ may be bonded together to form a cyclic group along with an adjacent phosphorus atom, (4) an optionally substituted amidino group, or (5) an optionally substituted guanidino group.

2. The composition according to claim 1, wherein the medical compound is (S)-(-)-8-[4-(2-butoxyethoxy)phenyl]-1-isobutyl-N-[4-[[(1-propyl-1H-imidazol-5-yl)methyl]sulfinyl]phenyl]-1,2,3,4-tetrahydro-1-benzazocine-5-carboxamide methanesulfonate.

3. The composition according to claim 1, wherein the surfactant is fatty acid glycerides having a polyoxyethylene chain as a hydrophilic group.

4. The composition according to claim 1, wherein the oily base is a glycerin fatty acid ester.

5. The composition according to claim 4, wherein the glycerin fatty acid ester is a glycerin tri-fatty acid ester.

6. The composition according to claim 5, wherein the glycerin tri-fatty acid ester is a glycerin tri-medium chain fatty acid ester.

7. The composition according to claim 6, wherein the glycerin tri-medium chain fatty acid ester is caprylic acid/capric acid triglycerides.

8. The composition according to claim 1, wherein the content of the surfactant is 10 w/w% to 90 w/w%.

9. The composition according to claim 1, wherein the content of the oily base is 1 w/w% to 50 w/w%.

10. A preparation comprising the composition according to any of claims 1 to 9.

11. The preparation according to claim 10, which is a capsule.

## Patentansprüche

1. Flüssige transparente pharmazeutische Zusammensetzung, umfassend:
(i) ein polares Lösungsmittel in einer Menge von 1 Gew.-% bis 20 Gew.-%, wobei es sich bei dem polaren Lösungsmittel um Wasser handelt;
(ii) eine ölige Grundlage;
(iii) ein Tensid mit einer HLB von 14 oder mehr, wobei der Gehalt (Gew.-%) des Tensids größer ist als der Gehalt (Gew.-%) der öligen Grundlage;
(iv) eine medizinische Verbindung mit einer Löslichkeit von weniger als 0,1 mg/ml in Wasser bei 25 °C, wobei der Gehalt der medizinischen Verbindung in der Zusammensetzung 1 Gew.-% oder mehr beträgt und wobei der Gehalt der medizinischen Verbindung einer Menge entspricht, die ihre Löslichkeit in dem Gemisch aus der öligen Grundlage und dem Tensid übersteigt, wobei die medizinische Verbindung ein Salz der durch Formel (I) dargestellten Verbindung ist: 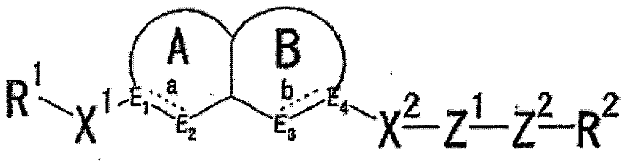
wobei R¹ einen gegebenenfalls substituierten fünf- bis sechsgliedrigen Ring bedeutet;
X¹ eine Bindung oder eine zweiwertige Gruppe bedeutet, wobei die Zahl der Atome, die die geradkettige Struktureinheit bilden, 1 bis 4 beträgt;
Ring A einen gegebenenfalls substituierten fünf- oder sechsgliedrigen Ring bedeutet und Ring B einen gegebenenfalls substituierten acht- bis zehngliedrigen Ring bedeutet;
E₁ und E₄ jeweils ein gegebenenfalls substituiertes Kohlenstoffatom oder ein gegebenenfalls substituiertes Stickstoffatom bedeuten;
E₂ und E₃ jeweils ein gegebenenfalls substituiertes Kohlenstoffatom, ein gegebenenfalls substituiertes Stickstoffatom, ein gegebenenfalls oxidiertes Schwefelatom oder ein Sauerstoffatom bedeuten;
a und b jeweils eine Einfachbindung oder eine Doppelbindung bedeuten;
X² eine zweiwertige Gruppe bedeutet, wobei die Zahl der Atome, die die geradkettige Struktureinheit bilden, 1 bis 4 beträgt;
Z¹ eine Bindung oder eine zweiwertige cyclische Gruppe bedeutet;
Z² eine Bindung oder eine zweiwertige Gruppe bedeutet;
R² Folgendes bedeutet: (1) eine gegebenenfalls substituierte Aminogruppe, wobei das Stickstoffatom zu einem quartären Ammonium oder Oxid umgewandelt sein kann, (2) eine gegebenenfalls substituierte stickstoffhaltige heterocyclische Gruppe, die Schwefelatome oder Sauerstoffatome als ringbildende Atome umfassen kann, wobei das Stickstoffatom zu einem quartären Ammonium oder Oxid umgewandelt sein kann, (3) eine Gruppe, die durch die Formel 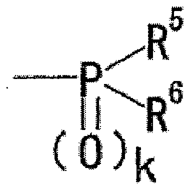
dargestellt wird, wobei k für 0 oder 1 steht und, wenn k = 0 ist, das Phosphoratom ein Phosphoniumsalz bilden kann, R⁵ und R⁶ jeweils eine gegebenenfalls substituierte Kohlenwasserstoffgruppe, gegebenenfalls substituierte Hydroxygruppe oder gegebenenfalls substituierte Aminogruppe bedeuten und R⁵ und R⁶ unter Bildung einer cyclischen Gruppe zusammen mit einem benachbarten Phosphoratom verbunden sein können, (4) eine gegebenenfalls substituierte Amidinogruppe oder (5) eine gegebenenfalls substituierte Guanidinogruppe.

2. Zusammensetzung gemäß Anspruch 1, wobei es sich bei der medizinischen Verbindung um (S)-(-)-8-[4-(2-Butoxyethoxy)phenyl]-1-isobutyl-N-[4-[[(1-propyl-1H-imidazol-5-yl)methyl]sulfinyl]phenyl]-1,2,3,4-tetra-hydro-1-benzazocin-5-carboxamidmethansulfonat handelt.

3. Zusammensetzung gemäß Anspruch 1, wobei es sich bei dem Tensid um Fettsäureglyceride mit einer Polyoxyethylenkette als hydrophiler Gruppe handelt.

4. Zusammensetzung gemäß Anspruch 1, wobei die ölige Grundlage ein Glycerinfettsäureester ist.

5. Zusammensetzung gemäß Anspruch 4, wobei der Glycerinfettsäureester ein Glycerintrifettsäureester ist.

6. Zusammensetzung gemäß Anspruch 5, wobei der Glycerintrifettsäureester ein Glycerintri(mittelkettige-Fettsäure)ester ist.

7. Zusammensetzung gemäß Anspruch 6, wobei es sich bei dem Glycerintri(mittelkettige-Fettsäure)ester um Caprylsäure-/Caprinsäuretriglyceride handelt.

8. Zusammensetzung gemäß Anspruch 1, wobei der Gehalt des Tensids 10 Gew.-% bis 90 Gew.-% beträgt.

9. Zusammensetzung gemäß Anspruch 1, wobei der Gehalt der öligen Grundlage 1 Gew.-% bis 50 Gew.-% beträgt.

10. Präparat, das die Zusammensetzung gemäß einem der Ansprüche 1 bis 9 umfasst.

11. Präparat gemäß Anspruch 10, bei dem es sich um eine Kapsel handelt.

## Revendications

1. Composition pharmaceutique transparente liquide comprenant
(i) un solvant polaire dans une quantité de 1 % p/p à 20 % p/p, où le solvant polaire est l'eau,
(ii) une base huileuse,
(iii) un tensioactif ayant une HLB de 14 ou plus, où la teneur (% p/p) du tensioactif est supérieure à la teneur (% p/p) de la base huileuse,
(iv) un composé médicinal ayant une solubilité de moins de 0,1 mg/mL dans l'eau à 25 °C, où la teneur du composé médicinal dans la composition est de 1 % p/p ou plus et où la teneur du composé médicinal est une quantité excédant sa solubilité dans le mélange de la base huileuse et du tensioactif, où le composé médicinal est un sel du composé représenté par la formule (I) : 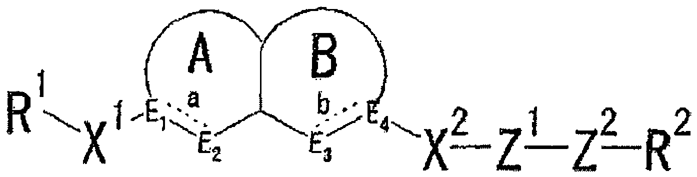
dans laquelle R¹ désigne un cycle à 5 ou 6 chaînons facultativement substitué,
X¹ désigne une liaison ou un groupe divalent, où le nombre d'atomes constituant le motif à chaîne droite est de 1 à 4,
le cycle A désigne un cycle à 5 ou 6 chaînons facultativement substitué, et le cycle B désigne un cycle à 8 à 10 chaînons facultativement substitué,
E₁ et E₄ désignent chacun un atome de carbone facultativement substitué ou un atome d'azote facultativement substitué,
E₂ et E₃ désignent chacun un atome de carbone facultativement substitué, un atome d'azote facultativement substitué, un atome de soufre facultativement oxydé ou un atome d'oxygène,
a et b désignent chacun une simple liaison ou une double liaison,
X² désigne un groupe divalent dans lequel le nombre d'atomes constituant le motif à chaîne droite est de 1 à 4,
Z₁ désigne une liaison ou un groupe cyclique divalent,
Z² désigne une liaison ou un groupe divalent,
R² désigne (1) un groupe amino facultativement substitué où l'atome d'azote peut être converti en un ammonium quaternaire ou un oxyde, (2) un groupe hétérocyclique facultativement substitué contenant de l'azote qui peut comprendre des atomes de soufre ou des atomes d'oxygène en tant qu'atomes constitutifs de cycle, où l'atome d'azote peut être converti en un ammonium quaternaire ou un oxyde, (3) un groupe représenté par la formule : 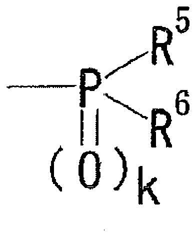
dans laquelle k désigne 0 ou 1, et lorsque k vaut 0, l'atome de phosphore peut former un sel de phosphonium, R⁵ et R⁶ désignent chacun un groupe hydrocarbure facultativement substitué, un groupe hydroxyle facultativement substitué ou un groupe amino facultativement substitué, et R⁵ et R⁶ peuvent être liés ensemble pour former un groupe cyclique conjointement avec un atome de phosphore adjacent, (4) un groupe amidino facultativement substitué, ou (5) un groupe guanidino facultativement substitué.

2. Composition selon la revendication 1, dans laquelle le composé médicinal est le méthanesulfonate de (S)-(-)-8-[4-(2-butoxyéthoxy)phényl]-1-isobutyl-N-[4-[[(1-propyl-1H-imidazol-5-yl)méthyl]sulfinyl]phényl]-1,2,3,4-tétrahydro-1-benzazocine-5-carboxamide.

3. Composition selon la revendication 1, dans laquelle le tensioactif est des glycérides d'acide gras ayant une chaîne poly(oxyéthylène) en tant que groupe hydrophile.

4. Composition selon la revendication 1, dans laquelle la base huileuse est un ester d'acide gras de glycérine.

5. Composition selon la revendication 4, dans laquelle l'ester d'acide gras de glycérine est un tri-ester d'acide gras de glycérine.

6. Composition selon la revendication 5, dans laquelle le tri-ester d'acide gras de glycérine est un tri-ester d'acide gras à chaîne moyenne de glycérine ;

7. Composition selon la revendication 6, dans laquelle le tri-ester d'acide gras à chaîne moyenne de glycérine est des triglycérides d'acide caprique/acide caprylique.

8. Composition selon la revendication 1, dans laquelle la teneur du tensioactif est de 10 % p/p à 90 % p/p.

9. Composition selon la revendication 1, dans laquelle la teneur de la base huileuse est de 1 % p/p à 50 % p/p.

10. Préparation comprenant la composition selon l'une quelconque des revendications 1 à 9.

11. Préparation selon la revendication 10, qui est une gélule.
